# EUROPÄISCHE PATENTSCHRIFT

(11) **EP 2 049 488 B1**
(45) Veröffentlichungstag und Bekanntmachung des Hinweises auf die Patenterteilung: **09.12.2015**
(21) Anmeldenummer: 07765125.5
(22) Anmeldetag: 07.07.2007
(51) Int. Cl.: C07D 213/57, C07D 213/61, A01N 43/40, A01N 47/40

(54) **N'-CYANO-N-HALOGENALKYL-IMIDAMID DERIVATE**
N'-CYANO-N-ALKYL HALIDE IMIDE AMIDE DERIVATIVES
DÉRIVÉS DE N'-CYANO-N-HALOGÈNALKYLE-IMIDAMIDE

(30) Priorität: 20.07.2006 DE 102006033572
(43) Veröffentlichungstag der Anmeldung: 22.04.2009
(73) Patentinhaber: Bayer Intellectual Property GmbH, 40789 Monheim am Rhein (DE)
(72) Erfinder: JESCHKE, Peter, 51467 Bergisch Gladbach (DE); VELTEN, Robert, 40764 Langenfeld (DE); FISCHER, Rüdiger, 50259 Pulheim (DE); HENSE, Achim, 51379 Leverkusen (DE); BECK, Michael, Edmund, 40789 Monheim (DE); MALSAM, Olga, 51503 Rösrath (DE); RECKMANN, Udo, 51063 Köln (DE); NAUEN, Ralf, 40764 Langenfeld (DE); GÖRGENS, Ulrich, 40882 Ratingen (DE); PITTA, Leonardo, 51371 Leverkusen (DE); SANWALD, Erich, 24159 Kiel (DE); ARNOLD, Christian, 40764 Langenfeld (DE)
(74) Vertreter: BIP Patents
(86) Internationale Anmeldenummer: PCT/EP2007/006044
(87) Internationale Veröffentlichungsnummer: WO 2008/009360

(56) Entgegenhaltungen:
- WO-A-91/04965
- WO-A-03/095418
- TOMIZAWA MOTOHIRO ET AL: "Neonicotinoid insecticide toxicology: Mechanisms of selective action" ANNUAL REVIEW OF PHARMACOLOGY AND TOXICOLOGY, ANNUAL REVIEW INC., PALO ALTO, CA, US, Bd. 45, 2005, Seiten 247-268, XP002461723 ISSN: 0362-1642

## Beschreibung

Die vorliegende Anmeldung betrifft neue substituierte *N'*-Cyano-*N*-halogenalkyl-imidamid Derivate, Verfahren zu Ihrer Herstellung und ihre Verwendung zur nichttherapeutischen Bekämpfung von tierischen Schädlingen, vor allem von Arthropoden, insbesondere von Insekten.

Bestimmte *N'*-Cyano-*N*-alkyl-imidamid Derivate sind bereits als Schädlingsbekämpfungsmittel bekannt geworden (vgl. WO 03/095418 A1). Darüber hinaus sind bestimmte *N'*-Cyano-*N-*monohalogenmethyl-imidamid Derivate als Schädlingsbekämpfungsmittel beschrieben (vgl. WO 91/04965 A1, T. Yamada, H. Takahashi, R. Hatano, In: Yamamoto I., Casida J. E. (Eds.), Neonicotinoid Insecticides and Nicotinic Acetylcholine Receptor, New York, pp. 149-175; P. Jeschke ChemBioChem 5, 570-589,2004).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Es wurden nun neue Verbindungen der Formel (I) gefunden, 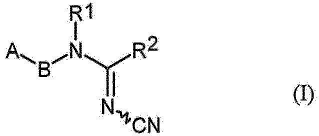 in welcher
- A: für 6-Chlor-pyrid-3-yl, 6-Brom-pyrid-3-yl, 6-Methyl-pyrid-3-yl, 6-Trifluormethyl-pyrid-3-yl, 2-Methyl-pyrimidin-5-yl, 2-Chlor-pyrimid-5-yl, für 1*H*-Pyrazol-4-yl, welches gegebenenfalls in 1-Position substituiert ist durch Methyl oder Ethyl, und in 3-Position durch Chlor, für 1*H*-Pyrazol-5-yl, 3-Methyl-pyrazol-5-yl, 2-Brom-1,3-thiazol-5-yl, 2-Chlor-1,3-thiazol-5-yl, für Isoxazol-5-yl, welches gegebenenfalls in 3-Position substituiert ist durch Methyl, Ethyl, Chlor oder Brom, 3-Methyl-1,2,4-oxadiazol-5-yl, 1-Methyl-1,2,4-triazol-3-yl oder 1,2,5-Thiadiazol-3-yl steht, oder worin
- A: für 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl oder 5-Methyl-6-brom-pyrid-3-yl steht, oder worin
- A: für einen Rest 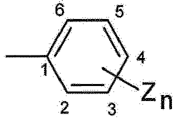 steht, in welchem
n für 2 oder 3 steht und
Z für Fluor oder Chlor steht,
- R¹: für 2,2-Difluorethyl steht,
- R²: für Methyl oder Ethyl steht und
- B: für einen Rest aus der Reihe (B-1) bis (B-9) 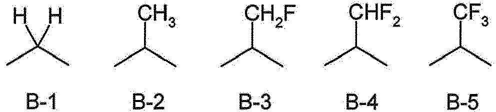 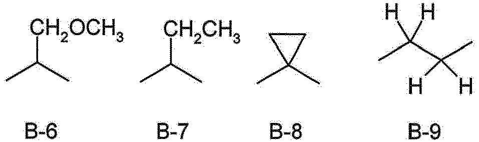 steht.

Ferner wurde gefunden, dass man die erfindungsgemäßen Verbindungen der Formel (I) erhält, wenn man
a) gemäß der Herstellungsmethode 1 Verbindungen der Formel (II) 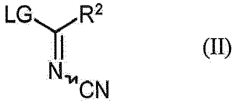 in welcher
   - R²: die oben genannte Bedeutung hat und LG für eine geeignete Abgangsgruppe (leaving group), beispielsweise C₁₋₂-alkoxy steht,
   in einem ersten Reaktionsschritt mit Verbindungen der Formel (III)

   R¹-NH₂ (III)

   in welcher
   - R¹: die oben genannte Bedeutung hat,
   gegebenenfalls in Gegenwart eines geeigneten Verdünnungsmittels und gegebenenfalls in Gegenwart eines basischen Hilfsmittels zu Verbindungen der Formel (IV) umsetzt, 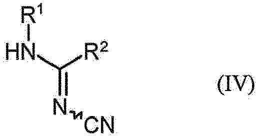 in welcher
   R¹ und R² die oben genannte Bedeutung haben,
   und diese dann in einem zweiten Reaktionsschritt mit Verbindungen der Formel (V)

   A-B-E (V)

   in welcher
   A und B die oben genannte Bedeutung haben,
   E für eine geeignete Abgangsgruppe (leaving group) LG wie z.B. Halogen insbesondere Brom, Chlor, Iod), O-Sulfonylalkyl oder O-Sulfonylaryl (insbesondere O-Mesyl, O-Tosyl) steht, gegebenenfalls in Gegenwart eines geeigneten Verdünnungsmittels und gegebenenfalls in Gegenwart eines basischen Hilfsmittels umsetzt, oder indem man
   - b): gemäß der Herstellungsmethode 2 Verbindungen der Formel (II) 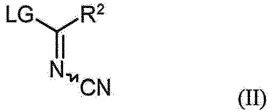 in welcher
   R² und LG die oben genannten Bedeutungen haben
   mit Verbindungen der Formel (VI)

   A-B-N(R¹)H (VI)

   in welcher
   A, B und R¹ die oben genannte Bedeutung haben,
   gegebenenfalls in Gegenwart eines geeigneten Verdünnungsmittels und gegebenenfalls in Gegenwart eines basischen Hilfsmittels umsetzt, oder indem man
   - c): gemäß der Herstellungsmethode 3 Orthoester der Formel (VII) 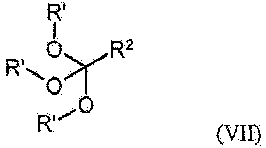 in welcher
   R² die oben genannte Bedeutung hat und
   R' für Methyl oder Ethyl steht in einem ersten Reaktionsschritt mit Cyanamid gegebenenfalls in Gegenwart von Verdünnungsmitteln *in-situ* zu Verbindungen der Formel (II) umsetzt 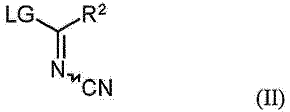 in welcher
   R² und LG die oben genannten Bedeutungen haben,
   und diese dann in einem zweiten Reaktionsschritt mit Verbindungen der Formel (VIII)

   A-B-NH₂ (VIII)

   in welcher
   A, und B die oben genannten Bedeutungen haben,
   gegebenenfalls in Gegenwart eines geeigneten Verdünnungsmittels und gegebenenfalls in Gegenwart eines basischen Hilfsmittels zu Verbindungen der Formel (IX) umsetzt, 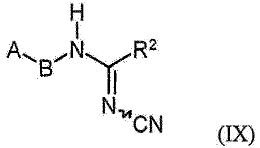 in welcher
   A, B, R² die oben genannte Bedeutungen haben,
   und diese dann in einem dritten Reaktionsschritt mit Verbindungen der Formel (X)

   R¹-E (X)

   in welcher
   R¹ und E die oben genannten Bedeutungen haben,
   gegebenenfalls in Gegenwart eines geeigneten Verdünnungsmittels und gegebenenfalls in Gegenwart eines basischen Hilfsmittels umsetzt.

Schließlich wurde gefunden, das die neuen Verbindungen der Formel (I) stark ausgeprägte biologische Eigenschaften besitzen und vor allem zur Bekämpfung von tierischen Schädlingen insbesondere von Insekten, Spinnentieren und Nematoden, die in der Landwirtschaft, in den Forsten, im Vorrats- und Materialschutz sowie auf dem Hygienesektor vorkommen geeignet sind.

Die Verbindungen der Formel (I) können gegebenenfalls in Abhängigkeit von der Art der Substituenten als geometrische und/oder als optisch Aktive Isomere oder entsprechende Isomeren-gemische in unterschiedlicher Zusammensetzung vorliegen. Die Erfindung betrifft sowohl die reinen Isomere als auch die Isomerengemische.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im Folgenden erläutert.
- A: steht bevorzugt für einen Rest aus der Reihe 6-Chlor-pyrid-3-yl, 6-Brom-pyrid-3-yl, 2-Methyl-pyrimidin-5-yl, 2-Chlor-pyrimid-5-yl, 3-Methyl-isoxazol-5-yl, 3-Brom-isoxazol-5-yl, 5-Fluor-6-chlor-pyrid-3-yl, 5,6-Dichlor-pyrid-3-yl, 5-Fluor-6-brom-pyrid-3-yl und 2-Chlor-1,3,-thiazol-5-yl.
- R¹: steht bevorzugt für 2,2-Difluorethyl.
- R²: steht bevorzugt für Methyl.
- B: steht bevorzugt für Methylen (-CH₂-).

In einer hervorgehobenen Gruppe von Verbindungen der Formel (I) stehen R² für Methyl, B für Methylen und A für 2-Chlor-pyrimidin-5-yl,

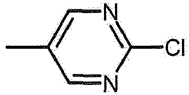

In einer weiteren hervorgehobenen Gruppe von Verbindungen der Formel (I) stehen R² für Methyl, B für Methylen und A für 5-Fluor-6-chlor-pyrid-3-yl,

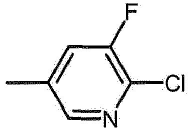

In einer weiteren hervorgehobenen Gruppe von Verbindungen der Formel (I) stehen R² für Methyl, B für Methylen und A für 5,6-Dichlor-pyrid-3-yl

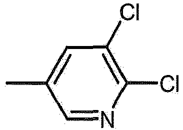

In einer weiteren hervorgehobenen Gruppe von Verbindungen der Formel (I) stehen R² für Methyl, B für Methylen und A für 5-Brom-6-chlor-pyrid-3-yl

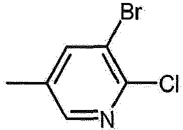

In einer weiteren hervorgehobenen Gruppe von Verbindungen der Formel (I) stehen R² für Methyl, B für Methylen und A für 5-Methyl-6-chlor-pyrid-3-yl

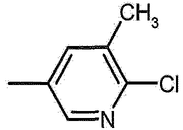

In einer weiteren hervorgehobenen Gruppe von Verbindungen der Formel (I) stehen R² für Methyl, B für Methylen und A für 5-Fluor-6-brom-pyrid-3-yl

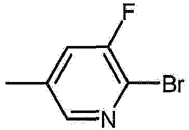

In einer weiteren hervorgehobenen Gruppe von Verbindungen der Formel (I) stehen R² für Methyl, B für Methylen und A für 5-Chlor-6-brom-pyrid-3-yl

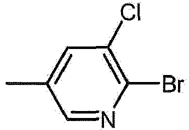

In einer hervorgehobenen Gruppe von Verbindungen der Formel (I) stehen R² für Methyl, B für Ethylen und A für 2-Chlor-pyrimidin-5-yl,

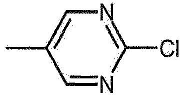

In einer weiteren hervorgehobenen Gruppe von Verbindungen der Formel (I) stehen R² für Methyl, B für Ethylen und A für 5-Fluor-6-chlor-pyrid-3-yl,

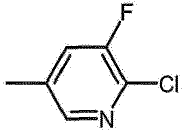

In einer weiteren hervorgehobenen Gruppe von Verbindungen der Formel (I) stehen R² für Methyl, B für Methylen und A für 5,6-Dichlor-pyrid-3-yl

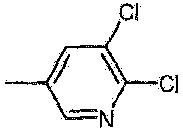

In einer weiteren hervorgehobenen Gruppe von Verbindungen der Formel (I) stehen R² für Methyl, B für Methylen und A für 5-Brom-6-chlor-pyrid-3-yl

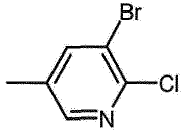

In einer weiteren hervorgehobenen Gruppe von Verbindungen der Formel (I) stehen R² für Methyl, B für Methylen und A für 5-Methyl-6-chlor-pyrid-3-yl

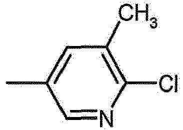

In einer weiteren hervorgehobenen Gruppe von Verbindungen der Formel (I) stehen R² für Methyl, B für Methylen und A für 5-Fluor-6-brom-pyrid-3-yl

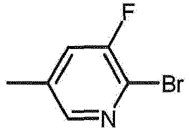

In einer weiteren hervorgehobenen Gruppe von Verbindungen der Formel (I) stehen R² für Methyl, B für Methylen und A für 5-Chlor-6-brom-pyrid-3-yl

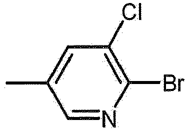

In einer hervorgehobenen Gruppe von Verbindungen der Formel (I) stehen R² für Methyl, B für Methylen und A für 2-Chlor-pyrimidin-5-yl,

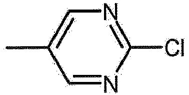

In einer weiteren hervorgehobenen Gruppe von Verbindungen der Formel (I) stehen R² für Methyl, B für Methylen und A für 5-Fluor-6-chlor-pyrid-3-yl,

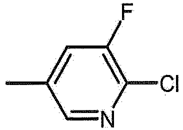

In einer weiteren hervorgehobenen Gruppe von Verbindungen der Formel (I) stehen R² für Methyl, B für Methylen und A für 5,6-Dichlor-pyrid-3-yl

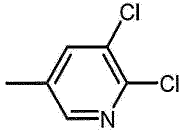

In einer weiteren hervorgehobenen Gruppe von Verbindungen der Formel (I) stehen R² für Methyl, B für Methylen und A für 5-Brom-6-chlor-pyrid-3-yl

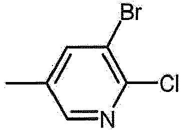

In einer weiteren hervorgehobenen Gruppe von Verbindungen der Formel (I) stehen R² für Methyl, B für Methylen und A für 5-Methyl-6-chlor-pyrid-3-yl

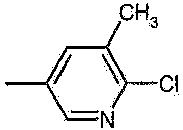

In einer weiteren hervorgehobenen Gruppe von Verbindungen der Formel (I) stehen R² für Methyl, B für Methylen und A für 5-Fluor-6-brom-pyrid-3-yl

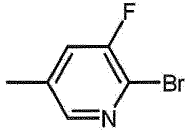

In einer weiteren hervorgehobenen Gruppe von Verbindungen der Formel (I) stehen R² für Methyl, B für Methylen und A für 5-Chlor-6-brom-pyrid-3-yl

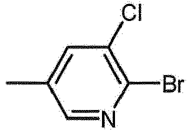

In einer weiteren hervorgehobenen Gruppe von Verbindungen der Formel (I) stehen R¹ für 2,2-Difluorethyl, R² für Methyl und B für Methylen.

In einer weiteren hervorgehobenen Gruppe von Verbindungen der Formel (I) stehen R¹ für 2,2-Difluorethyl, R² für Ethyl und B für Methylen.

Die oben aufgeführten allgemeinen oder im Vorzugsbereich aufgeführten Restedefmitionen bzw. Erläuterungen gelten für die Endprodukte und für die Ausgangsprodukte und Zwischenprodukte entsprechend. Diese Restedefinitionen können untereinander also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Setzt man bei dem erfindungsgemäßen Verfahren 1 zur Herstellung der neuen Verbindungen der allgemeinen Formel (I) im ersten Reaktionsschritt als Verbindungen der Formel (II) beispielsweise *N*-Cyan-ethanimidsäuremethylester und als Verbindungen der Formel (III) beispielsweise 2,2-Difluorethanamin ein und lässt die auf diese Weise entstandenen Verbindungen der allgemeinen Formel (IV), beispielsweise das N'-Cyan-N-(2,2-difluorethyl)ethanimidamid, mit Verbindungen der allgemeinen Formel (V), beispielsweise das 6-Chlor-3-chlormethyl-pyridin (CCMP), reagieren, so lässt sich das Herstellungsverfahren 1 durch folgendes Reaktionsschema I wiedergeben:

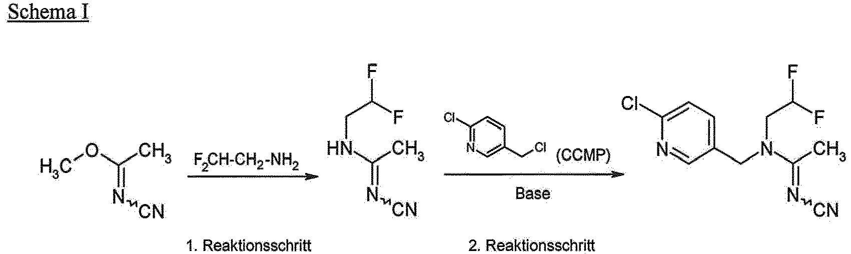

Die im ersten Reaktionsschritt des erfindungsgemäßen Verfahrens 1 als Ausgangsstoffe benötigten Verbindungen sind durch die Formel (II) allgemein definiert.

In dieser Formel (II) steht R² vorzugsweise für diejenigen Reste, die bereits im Zusammenhang mit der Beschreibung der erfindungsgemäßen Stoffe der allgemeinen Formel (I) als bevorzugte Substituenten genannt wurden.

Die Verbindungen der Formel (II) sind bekannt (vgl. z.B. R²=Me und Et, LG=OMe: B. Arnold, M. Regitz Tetrahedron Lett. (1980), 21, 909-912; A. A. Perez et al. Synthesis (1983), 5, 402-404; R²=Me, LG=OEt: W. Lwowski, Synthesis 1971, 5, 263, DE 3 411203 C1 (1985); R²=Et, LG=OEt: US 4 734 413 A (1988); R²=n-Pr, LG=OMe: WO 93/00341 A1 (1993); R²=*iso*-Pr, LG=OMe: WO 19990617 (1999); R²=*cyc*/*o*-Pr, LG=OMe: US 4670559 (1988) oder können nach bekannten Verfahren hergestellt warden.

Beispielsweise lassen sich Orthoester (vgl. H. Schäfer, K. Gewald, J. Prakt. Chem. 1976, 318, 347-349) der Formel (VII), wie im nachfolgenden Herstellungsverfahren 3 beschrieben, nach literaturbekannten Methoden mit Cyanamid zu Verbindungen der Formel (II) umsetzen (LG = OR'; Reaktionsschema II; vgl. auch das weiter unten genannte Herstellungsverfahren 3)

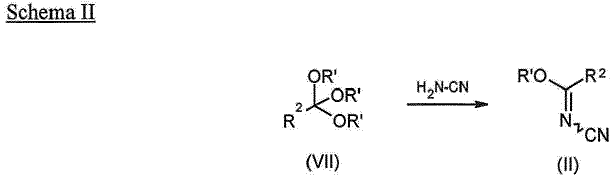

Die weiterhin zur Durchführung des ersten Reaktionsschrittes im erfindungsgemäßen Verfahrens 1 als Ausgangsstoffe zu verwendenden Verbindungen sind allgemein durch die Formel (III) definiert.

In der Formel (III) hat R¹ die Bedeutung die bereits im Zusammenhang mit der Beschreibung der erfindungsgemäßen Stoffe der Formel (I) genannt wurde.

Die Aminoverbindungen der Formel (III) sind kommerziell erhältlich oder können in an sich bekannter Weise beispielsweise nach der "Leuckart-Wallach-Reaktion (Verbindungen der allgemeinen Formel (III) in welcher R¹ für Alkyl steht, primäre Amine: vgl. z.B. Houbel-Weyl, Methoden der Organischen Chemie, Bd. XI/1, 4. Aufl. 1957, G. Thieme Verlag, Stuttgart, S. 648; M. L. Moore in "The Leuckart Reaction" in: Organic Reactions, Bd. 5, 2. Aufl. 1952, New York, John Wiley & Sons, Inc. London) erhalten werden. Das im ersten Reaktionsschritt verwendete 2,2-Difluorethylamin (vgl. US 4030994 (1977) und das entsprechende Hydrochlorid (A. Donetti et al., J. Med. Chem. 1989,32,957-961) sind bekannt.

Die im zweiten Reaktionsschritt des erfindungsgemäßen Verfahrens 1 als Ausgangsstoffe benötigten Verbindungen sind durch die Formel (V) allgemein definiert.

In dieser Formel (V) stehen A und B vorzugsweise für diejenigen Reste, die bereits im Zusammenhang mit der Beschreibung der erfindungsgemäßen Stoffe der Formel (I) als bevorzugte Substituenten genannt werden.

E steht für eine geeignete Abgangsgruppe, wie weiter oben beschrieben.

Allgemeine Wege zur Herstellung der Ausgangsverbindungen (A-1), worin B für eine Gruppe -C(R³R⁴)- steht und E eine geeignete Abgangsgruppe LG (= leaving group) bedeutet, sind im Reaktionsschema III wiedergegeben.

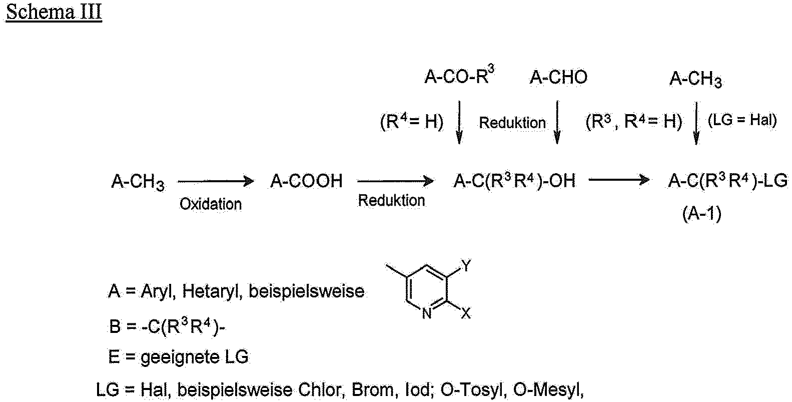

Die Verbindungen (A-1, R³, R⁴ = Wasserstoff) sind teilweise bekannt oder können nach bekannten Methoden erhalten werden (z.B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5, 257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 2-Methyl-3-chlormethyl-pyridin: EP 302 389 A2 (1989); 2-Trifluormethyl-3-chlormethyl-pyridin: WO 2004082616 A2 (2004); 3-Chlor-6-chlormethyl-pyridazin: EP 284 174 A1 (1988); 2-Chlor-5-pyrazinylmethylchlorid: J. Heterocycl. Chem. 23,149-151 (1986) 2-Chlor-5-pyrazinylmethylbromid: JP 05 239 034 A2 (1993);

Methylsubstituierte Aromaten oder Heterocyclen der Formel A-CH₃ lassen sich beispielsweise durch Oxidation in entsprechende aromatische oder heterocyclische Carbonsäuren (A-COOH, z.B. 5-Fluor-6-brom-nicotinsäure: F. L. Setliff, G. O. Rankin, J. Chem. Eng. Data (1972), 17, 515-516; 5-Chlor-6-brom-nicotinsäure und 5,6-Dibrom-nicotinsäure: F. L. Setliff et al., J. Chem. Eng. Data (1981), 26, 332-333; 5-Iod-6-brom-nicotinsäure: F. L. Setliff et al., J. Chem. Eng. Data (1978), 23, 96-97, 5-Fluor-6-iod-nicotinsäure und 5-Brom-6-iod-nicotinsäure: F. L. Setliff et al., J. Chem. Eng. Data (1973), 18, 449-450, 5-Chlor-6-iod-nicotinsäure: F. L. Setliff, J. E. Lane J. Chem. Eng. Data (1976), 21, 246-247) oder Carbonsäureester (z. B. 5-Methyl-6-fluor-nicotinsäuremethylester: WO 9833772 A1, 1998; 5-Methyl-6-brom-nicotinsäure-methylester: WO 9730032 A1, 1997) überführen. Weiterhin ist die Synthese von formylsubstituierten Aromaten oder Heterocyclen (A-CHO, z. B. 6-Chlor-3-formyl-5-methyl-pyridin: DE 4429465 A1, 1996) aus nicht cyclischen Ausgangskomponenten vorbeschrieben; diese kann beispielsweise mittels 1,3-dipolarer Cycloaddition (z. B.: 5-Chlormethyl-3-brom-isoxazol: P. Pevarello, M. Varasi Synth. Commun. (1992), 22, 1939-1948) erfolgen.

Die aromatischen oder heterocyclischen Carbonsäuren (A-COOH) oder Alkylcarbonylverbindungen (A-CO-R³; R³ = Alkyl) können dann nach literaturbekannten Methoden in die entsprechenden aromatischen oder heterocyclischen Hydroxyalkylverbindungen (A-C(R³R⁴)-OH; R³ = H, Alkyl; R⁴ = H) überführt werden, die anschließend nach literaturbekannten Methoden zu aktivierten aromatischen oder heterocyclischen Hydroxymethylverbindungen (A-C(R³R⁴)-LG, LG = OTosyl, OMesyl) bzw. aromatischen oder heterocyclischen Halogenmethylverbindungen (A-C(R³R⁴)-LG, LG = Hal) umgesetzt werden. Letztere können auch aus entsprechenden methylsubstituierten Aromaten oder Heterocyclen der Formel A-CH₃ unter Verwendung von geeigneten und literaturbekannten Halogenierungsmitteln erhalten werden. Als Beispiele für diese Vorgehensweise seien die Synthesen der Halogenmethyl-substituierten Heterocyclen genannt: 5-Chlormethyl-2-methyl-pyrimidin (U. Eiermann et al., Chem. Ber. (1990), 123, 1885-9); 3-Chlomethyl-5-brom-6-chlor-pyridin, 3-Brom-5-iod-6-chlor-pyridin (S. Kagabu et al., J. Pestic. Sci. (2005), 30, 409-413).

Ausgangsverbindungen (A-7) in denen A für einen 5,6-disubstituierten Pyrid-3-yl Rest steht, können ebenso nach literaturbekannten Methoden erhalten werden. Beispielsweise sind geeignete und literaturbekannte Ausgangsverbindungen die 6-Halogen-substituierten 5-Nitro-β-picoline (A-2), die entsprechend bekannter Literaturvorschriften modifiziert werden können, wie im Reaktionsschema IV gezeigt.

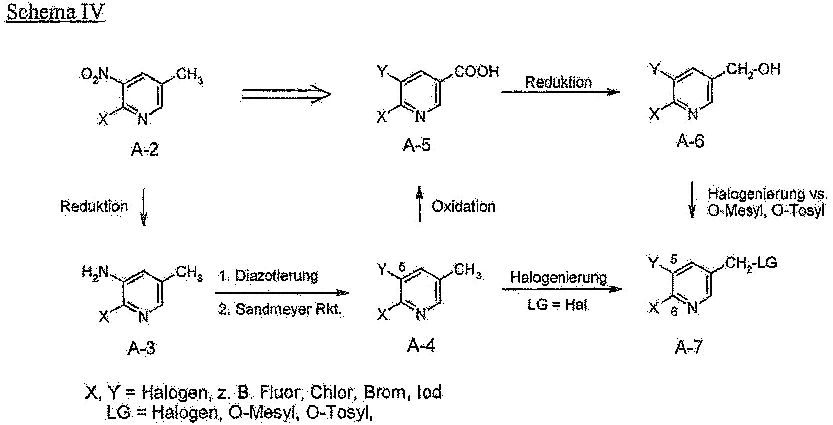

Beispielsweise führt die Reduktion der Nitrogruppe in 6-Halogen-substituierten 5-Nitro-β-picolinen (A-2) zu 6-Halogen-substituierten 5-Amino-β-picolinen (A-3, z. B. 5-Amino-6-chlor-β-picolin und 5-Amino-6-brom-β-picolin: Setliff, F. L. Org. Preparations and Preparations Int. (1971), 3, 217-222; Kagabu, S. et al. J. Pestic. Sci. (2005), 30, 409-413). Durch nachfolgende Diazotierung sowie Sandmeyer-Reaktion (C. F. H. Allen, J. R. Thirtle, Org. Synth., Coll. Vol. III, 1955, S. 136) ist die Einführung von Halogensubstituenten in 5-Position möglich (A-4, z. B. 5-Fluor-6-chlor-β-picolin und 5-Fluor-6-brom-β-picolin: Setliff, F. L. Org. Preparations and Preparations Int. (1971), 3, 217-222; 5-Iod-6-chlor-β-picolin: Kagabu, S. et al. J. Pestic. Sci. (2005), 30, 409-413; 5,6-Dichlor-picolin: Setliff, F. L.; Lane, J. E. J. Chem. Engineering Data (1976), 21, 246-247). Die Oxidation der Methylgruppe in den 5,6-disubstituierten β-Picolinen (A-4) führt dann zu den entsprechenden 5,6-disubstituierten Nicotinsäuren (A-5, z. B. 5-Fluor-6-chlor-nicotinsäure und 5-Fluor-6-brom-nicotinsäure: Setliff F. L., Rankin G. O. J. Chem. Engineering Data (1972), 17, 515-516; 5-Brom-6-fluor-nicotinsäure, 5-Brom-6-chlor-nicotinsäure und 5-Brom-6-brom-nicotin-säure: F. L. Setliff J. Chem. Engineering Data (1970), 15, 590-591; 5-Chlor-6-brom-nicotinsäure und 5-Iod-6-brom-nicotinsäure: Setliff, F. L., Greene, J. S. J. Chem. Engineering Data (1978), 23, 96-97; bekannt ist auch die 5-Chlor-6-trifluormethyl-nicotinsäure: F. Cottet et al., Synthesis (2004), 10, 1619-1624), die in Gegenwart von Reduktionsmitteln zu den entsprechenden hydroxymethylierten Pyridinen (A-6) umgewandelt werden können (z. B. 5-Brom-6-chlor-3-hydroxy-methyl-pyridin: Kagabu, S. et al., J. Pestic. Sci. (2005), 30, 409-413).

Durch Verwendung von 6-Chlor-5-nitro-nicotinsäure (A-5, X=Cl, Y=NO₂; Boyer, J. H.; Schoen, W., J. Am. Chem. Soc. (1956), 78, 423-425) kann mittels Reduktion das 6-Chlor-3-hydroxymethyl-5-nitro-pyridin (A-6, X=Cl, Y=NO₂; Kagabu, S. et al., J. Med. Chem. (2000), 43, 5003-5009) gebildet werden, das nachfolgend zum 6-Chlor-3-hydroxymethyl-5-amino-pyridin (A-6, X=Cl, Y=NH₂; Kagabu, S. et al., J. Med. Chem. (2000), 43, 5003-5009) reduziert und über Diazotierung und Reaktion mit Hydroxylamin in das 6-Chlor-3-hydroxymethyl-5-azido-pyridin (A-6, X=Cl, Y=N₃; Kagabu, S. et al., J. Med. Chem. (2000), 43, 5003-5009) überführt wird. Nachfolgende Halogenierung mit Thionylchlorid ergibt das 6-Chlor-3-chlormethyl-5-azido-pyridin (A-7, X=Cl, Y=N₃, LG=Cl; Kagabu, S. et al., J. Med. Chem. (2000), 43, 5003-5009).

Alternativ führt die Halogenierung der Methylgruppe in 3-Position von (A-4) zu den Verbindungen (A-7), in der LG für Halogen steht (z.B.: 3-Brommethyl-6-chlor-5-fluor-pyridin, 3-Brommethyl-6-chlor-5-iod-pyridin: Kagabu, S. et al. J. Pestic. Sci. (2005), 30, 409-413). Bei Verwendung von 6-Halogen-substituierten 5-Nitro-β-picolinen (A-4; Y=NO₂) kann hierbei zunächst die Halogenierung der Methylgruppe in 3-Position erfolgen (z. B. 3-Brommethyl-6-chlor-5-nitro-pyridin: Kagabu, S. et al., J. Pestic. Sci. (2005), 30, 409-413). Die Nitrogruppe kann auch gegebenenfalls erst zu einem späteren Zeitpunkt in der Reaktionssequenz reduziert werden.

Literaturbekannt ist ebenso die Einführung von Substituenten in 5-Position (z. B. Y=N₃) bei Verbindungen (A-7), in der LG für *N*-Morpholino steht. Dieser Rest kann anschließend sehr einfach durch Halogen (LG=Hal) ersetzt werden (vgl. S. Kagabu et al., J. Med. Chem. 2000, 43, 5003-5009; Reaktionsbedingungen: Chlorameisensäureethylester, Tetrahydrofuran, 60°C).

Im Allgemeinen gelingt es, Halogenatome in Nachbarschaft zum Pyridinstickstoff durch andere Halogenatome oder halogenierte Gruppen, beispielsweise Trifluormethyl, zu ersetzen (Transhalogenierung, z.B.: Chlor gegen Brom oder Iod; Brom gegen Iod oder Fluor; Iod gegen Fluor oder Trifluormethyl). Deshalb besteht ein weiterer alternativer Syntheseweg darin, das Halogenatom (beispielsweise X=Cl) in 6-Position des Pyrid-5-yl-restes (z.B. in A-5 mit X, Y=Cl; 5,6-Dichlornicotinsäure: Setliff, F. L.; Lane, J. E. J. Chem. Engineering Data (1976), 21, 246-247) gegen ein anderes Halogenatom, beispielsweise Iod oder Fluor, auszutauschen (z.B.: A-5 mit X=I; 5-Brom-6-iod-nicotinsäure und A-5 mit X=F; 5-Brom-6-fluor-nicotinsäure: Setliff, F. L.; Price, D. W. J. Chem. Engineering Data (1973), 18, 449-450) zu ersetzen. Diese Transhalogenierung kann aber auch gegebenenfalls erst in geeigneten Verbindungen der Formel (I) erfolgen.

Im Allgemeinen ist es vorteilhaft, das erfindungsgemäße Herstellungsverfahren 1 gegebenenfalls in Gegenwart von Verdünnungsmitteln und gegebenenfalls in Gegenwart basischer Reaktionshilfsmittel durchzuführen.

Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 1 kommen alle inerten organischen Lösungsmittel in Frage.

Als Beispiele sind zu nennen: Halogenkohlenwasserstoffe, insbesondere Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methylmorpholin, Pyridin und Tetramethylendiamin; Nitrokohlenwasserstoffe wie Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Chlorbenzol, Brombenzol, Nitro-benzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N*-Methyl-formamid, N,N-Dimethyl-formamid, *N*,*N*-Dipropyl-formamid, *N*,*N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N*,*N'*-1,4-Diformyl-piperazin; Ketone wie Aceton, Acetophenon, Methylethylketon, Methylbutylketon.

Selbstverständlich kann man in das erfindungsgemäße Verfahren auch Gemische der genannten Lösungs- und Verdünnungsmittel einsetzen.

Bevorzugte Verdünnungsmittel zur Durchführung des ersten Reaktionsschrittes des erfindungsgemäßen Herstellungsverfahrens 1 sind jedoch Alkohole wie Methanol, Ethanol, Isopropanol oder Butanol.

Bevorzugte Verdünnungsmittel zur Durchführung des zweiten Reaktionsschrittes des erfindungsgemäßen Herstellungsverfahrens 1 sind Nitrile wie Acetonitril, Propionitril, Butyronitril, Isobutyronitril, Benzonitril oder m-Chlorbenzonitril, insbesondere wird Acetonitril verwendet.

Als basische Reaktionshilfsmittel zur Durchführung des zweiten Reaktionsschrittes des erfindungsgemäßen Herstellungsverfahrens 1 können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Hydride, Oxide, Hydrogencarbonate, Carbonate des Lithiums, Natriums, Kaliums, Cäsiums, Magnesiums, Calciums und Bariums, ferner weitere basische Verbindungen wie Amidinbasen oder Guanidinbasen wie 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), N,N,N,N-Tetramethyl-l,8-naphthalindiamin, Pentamethylpiperidin, tertiäre Amine wie Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, N,N,N',N'-Tetramethylendiamin, N,N',N'-Tetraethylendiamin, Chinoxalin, N-Propyl-diisopropylamin, N-Ethyl-diisopropylamin, N,N'-Dimethyl-cyclohexylamin, 2,6-Lutidin, 2,4-Lutidin oder Triethyldiamin.

Vorzugsweise findet Salze des Cäsiums, beispielsweise Cäsiumcarbonat oder Casiumiodid, Verwendung.

Die Umsetzung von Verbindungen der Formel (II) nach dem ersten Reaktionsschritt des Herstellungsverfahren 1 wird durchgeführt indem man die Verbindungen der Formel (II) in Gegenwart von Verbindungen der Formel (III) in einem der angegebenen Verdünnungsmittel umsetzt.

Die Reaktionsdauer beträgt 5 Minuten bis 48 Stunden. Die Umsetzung erfolgt bei Temperaturen zwischen -100°C und +200°C, bevorzugt zwischen -50°C und 150°C, besonders bevorzugt bei Raumtemperatur.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des ersten Reaktionsschrittes des erfindungsgemäßen Herstellungsverfahrens 1 setzt man pro Mol Verbindung der allgemeinen Formel (II) im allgemeinen 0,5 bis 4,0 Mol, vorzugsweise 0,7 bis 3,0 Mol, besonders bevorzugt 1,0 bis 2,0 Mol an Verbindungen der Formel (III) ein.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Umsetzung von Verbindungen der Formel (IV) nach dem zweiten Reaktionsschritt des Herstellungsverfahren 1 wird durchgeführt, indem man die Verbindungen der allgemeinen Formel (IV) in Gegenwart von Verbindungen der Formel (V) in einem der angegebenen Verdünnungsmittel und in Gegenwart eines basischen Reaktionshilfsmittels umsetzt.

Die Reaktionsdauer beträgt 5 Minuten bis 48 Stunden. Die Umsetzung erfolgt bei Temperaturen zwischen -100°C und +200°C, bevorzugt zwischen -50°C und 150°C, besonders bevorzugt bei Rückflusstemperatur des weiter oben angegebenen Lösungsmittels.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des zweiten Reaktionsschrittes des erfindungsgemäßen Herstellungsverfahrens 1 setzt man pro Mol Verbindung der Formel (IV) im allgemeinen 0,5 bis 4,0 Mol, vorzugsweise 0,7 bis 3,0 Mol, besonders bevorzugt 1,0 bis 2,0 Mol an Verbindungen der Formel (V) ein.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Setzt man bei dem erfindungsgemäßen Herstellungsverfahren 2 zur Herstellung der neuen Verbindungen der Formel (I) als Verbindungen der Formel (II) beispielsweise *N*-Cyan-ethanimidsäuremethylester, mit Verbindungen der Formel (VI), beispielsweise mit N-[(6-chlor-5-fluorpyridin-3-yl)methyl]-2,2-difluorethylamin um, so kann das Herstellungsverfahren 2 durch das Reaktionsschema V wiedergeben werden:

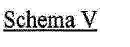

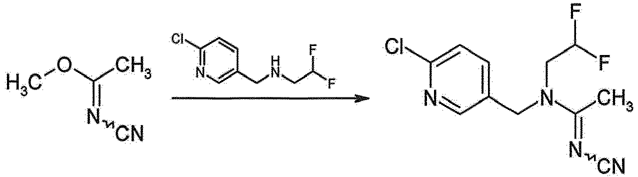

Die im erfindungsgemäßen Verfahren 2 als Ausgangsstoffe benötigten Verbindungen sind durch die Formel (II) allgemein definiert.

In dieser Formel (II) steht R² vorzugsweise für diejenigen Reste, die bereits im Zusammenhang mit der Beschreibung der erfindungsgemäßen Stoffe der allgemeinen Formel (I) als bevorzugte Substituenten genannt werden.

Die Verbindungen der allgemeinen Formel (II) können nach dem weiter oben beschriebenen Herstellungsverfahren 1 aus Orthoester und Cyanamid erhalten werden (vgl. Schema II).

Die weiterhin zur Durchführung des erfindungsgemäßen Verfahrens 2 als Ausgangsstoffe zu verwendenden Verbindungen sind allgemein durch die Formel (VI) definiert.

In der Formel (VI) haben A, B und R¹ die Bedeutung die bereits im Zusammenhang mit der Beschreibung der erfindungsgemäßen Stoffe der allgemeinen Formel (I) für Substituenten genannt wurde.

Zur Darstellung der Ausgangsverbindungen (A-8) worin B für C(R³R⁴) steht, ist es vorteilhaft, dass man beispielsweise Verbindungen der Formel (A-1), in der A die weiter oben genannte Bedeutung hat und LG für eine gegeignete Abgangsgruppe steht (z. B. Chlor, Brom, Iod, O-Tosyl, O-Mesyl), mit Verbindungen der allgemeinen Formel (A-9), in der R' für Halogen-haltiges Alkyl steht, gegebenenfalls in Gegenwart von Verdünnungsmitteln und gegebenenfalls in Gegenwart von den im Herstellungsverfahren 1 genannten basischen Reaktionshilfsmitteln umsetzt (vgl. Reaktionsschema VI).

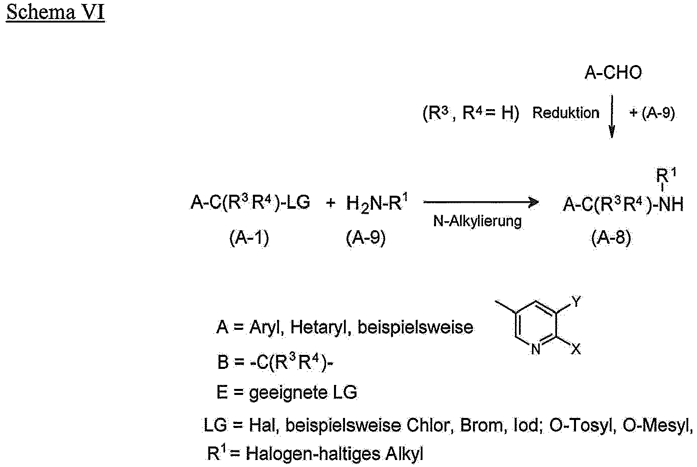

Alternativ und in bestimmten Fällen ist aber auch eine Herstellung von Ausgangsverbindungen (A-8), in der R³ und R⁴ für Wasserstoff stehen, aus den entsprechenden Aldehyden (A-CHO) und den Verbindungen (A-9) mittels reduktiver Aminierung möglich (vgl. Houben-Weyl, Methoden der Organischen Chemie, Bd. XI/1, Seite 602, G. Thieme Verlag, Stuttgart).

Im Allgemeinen ist es vorteilhaft, das erfindungsgemäße Herstellungsverfahren 2 gegebenenfalls in Gegenwart eines weiter oben genannten Verdünnungsmittels durchzuführen.

Die Umsetzung von Verbindungen der Formel (II) nach dem Herstellungsverfahren 2 wird durchgeführt, indem man diese mit Verbindungen der Formel (VI) umsetzt.

Die Reaktionsdauer beträgt 5 Minuten bis 48 Stunden. Die Umsetzung erfolgt bei Temperaturen zwischen -100°C und +200°C, bevorzugt zwischen -50°C und 150°C.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des erfindungsgemäßen Herstellungsverfahrens 2 setzt man pro Mol Verbindung der Formel (II) im allgemeinen 0,5 bis 4,0 Mol, vorzugsweise 0,7 bis 3,0 Mol, besonders bevorzugt 1,0 bis 2,0 Mol an Verbindungen der Formel (VII) ein.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Setzt man bei dem erfindungsgemäßen Herstellungsverfahren 3 zur Herstellung der neuen Verbindungen der Formel (I) im ersten Reaktionsschritt als Verbindungen der Formel (VII) beispielsweise Orthoessigsäuremethylester in Gegenwart von Cyanamid ein und lässt die *in-situ* gebildeten Verbindungen der Formel (II), beispielsweise den *N*-Cyan-ethanimidsäuremethylester, in einem zweiten Reaktionsschritt mit Verbindungen der Formel (VIII), beispielsweise mit 3-Aminomethyl-6-chlor-pyridin zu Verbindungen der Formel (IX), beispielsweise dem N'-Cyano-N-[6-chlor-pyrid-3-ylmethyl]ethanimidamid reagieren, und N-alkyliert diesen im dritten Reaktionsschritt in Gegenwart von Verbindungen der Formel (X), beispielsweise mit 2,2-Difluor-ethylbromid, so kann das Herstellungsverfahren 3 durch das Reaktionsschema VII wiedergeben werden:

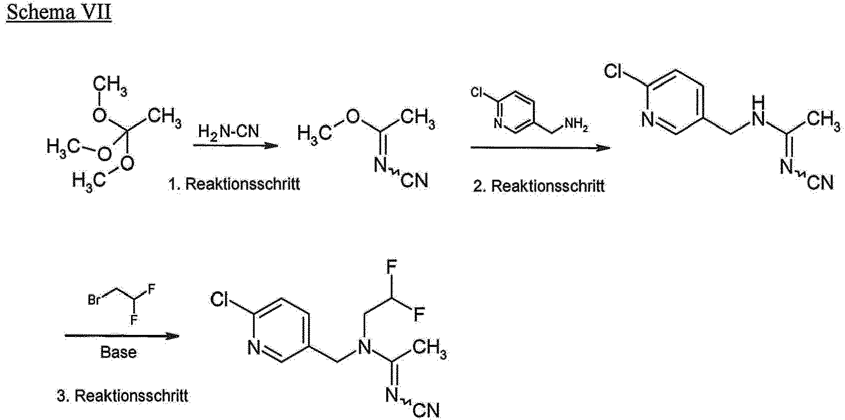

Die im erfindungsgemäßen Verfahren 3 als Ausgangsstoffe benötigten Verbindungen sind durch die Formel (VII) allgemein definiert.

In dieser Formel (VII) steht R² vorzugsweise für diejenigen Reste, die bereits im Zusammenhang mit der Beschreibung der erfindungsgemäßen Stoffe der allgemeinen Formel (I) als bevorzugte Substituenten genannt werden. R' steht in dieser Formel (VII) vorzugsweise für C₁-C₄-Alkyl, insbesondere für Methyl oder Ethyl.

Die Verbindungen der Formel (VII) und Cyanamid sind bekannte Verbindungen (vgl. auch H. Schäfer, K. Gewald, J. Prakt. Chem. 1976, 318, 347-349).

Die weiterhin zur Durchführung des ersten Reaktionsschrittes im erfindungsgemäßen Verfahren 3 als Ausgangsstoffe zu verwendenden Verbindungen sind allgemein durch die Formel (VIII) definiert.

In der Formel (VIII) haben A und B die Bedeutung die bereits im Zusammenhang mit der Beschreibung der erfindungsgemäßen Stoffe der allgemeinen Formel (I) für Substituenten genannt wurde.

Der ersten beiden Reaktionsschritte des erfindungsgemäßen Herstellungsverfahrens 3 können in Analogie zu H. Schäfer, K. Gewald, J. Prakt. Chem. 1976, 318, 347-349 (Synthese von Arylaminoethylen-cyanamiden) und zu dem Ausführungsbeispiel 1 in WO 03/095418 A1 (N'-Cyano-N-aryl-ethyl)propanimidamide) durchgeführt werden Bevorzugt wird ein Eintopfverfahren, das gegebenenfalls ohne Gegenwart eines Verdünnungsmittel (in Abhängigkeit des jeweiligen Orthoesters) durchgeführt werden kann.

Die Umsetzung von Verbindungen der Formel (II) gemäß den ersten beiden Reaktionsschritten nach dem Herstellungsverfahren 3 wird durchgeführt, indem man die Verbindungen der Formel (VI) in Gegenwart von Cyanamid und von Verbindungen der Formel (VIII) umsetzt.

Die Reaktionsdauer beträgt 5 Minuten bis 48 Stunden. Die Umsetzung erfolgt bei Temperaturen zwischen -100°C und +200°C, bevorzugt zwischen -50°C und 150°C, besonders bevorzugt bei der Siedetemperatur des jeweiligen Orthoesters.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des erfindungsgemäßen Herstellungsverfahrens 3 setzt man pro Mol Verbindung der allgemeinen Formel (II) im allgemeinen 0,5 bis 4,0 Mol, vorzugsweise 0,7 bis 3,0 Mol, besonders bevorzugt 1,0 bis 2,0 Mol an Cyanamid und Verbindungen der allgemeinen Formel (VIII) ein.

Nach vollendeter Umsetzung wird der gesamte Reaktionsansatz eingeengt. Die nach Aufarbeitung anfallenden Verbindungen der Formel (IX) lassen sich in üblicher Weise durch Umkristallisieren, Vakuumdestillation oder Säulenchromatographie reinigen (vgl. auch die Herstellungsbeispiele).

Die weiterhin zur Durchführung des dritten Reaktionsschrittes im erfindungsgemäßen Verfahren 3 als Ausgangsstoffe zu verwendenden Verbindungen sind allgemein durch die Formel (X) definiert.

In der Formel (X) hat R¹ die Bedeutung, die bereits im Zusammenhang mit der Beschreibung der erfindungsgemäßen Stoffe der allgemeinen Formel (I) genannt wurde.

E steht für eine geeignete Abgangsgruppe, wie weiter oben beschrieben.

Die Verbindungen der Formel (X) können z.T. kommerziell oder nach literaturbekannten Methoden erhalten werden (vgl. Verbindungen der Formel (X) in welcher E für Halogen wie Chlor, Brom und Iod steht: Houben-Weyl Band Methoden der Organischen Chemie Band V/3, S. 503 und Band V/4 S. 13, 517, G. Thieme Verlag, Stuttgart; E¹ für Mesylat steht: Crossland, R. K., Servis, K. L. J. Org. Chem. (1970), 35, 3195; E für Tosylat steht: Roos, A. T. et al., Org. Synth., Coll. Vol. I, (1941), 145; Marvel, C. S., Sekera, V. C. Org. Synth., Coll. Vol. III, (1955), 366. Die verwendeten halogenierten Alkane sind beispielsweise literaturbekannt (vgl. R¹ = CH₂CHF₂, E = Br, 2,2-Difluorethylbromid: EP 420815; R¹ = CH₂CHF₂, E = I, 2,2-Difluorethyliodid: A. Kamal et al., Tetrahedron Lett. (2002), 43, 7353-7355), R¹ = CH₂CHF₂, E = OSO₂Me, Methansulfonsäure-2,2-difluorethylester: DE 4315371, WO 2002044145).

Im Allgemeinen ist es vorteilhaft, den dritten Reaktionsschritt des erfindungsgemäßen Herstellungsverfahrens 3 in Gegenwart von Verdünnungsmitteln durchzuführen und in Gegenwart von basischen Reaktionshilfsmitteln durchzuführen.

Bevorzugte Verdünnungsmittel zur Durchführung des dritten Reaktionsschrittes des erfindungsgemäßen Herstellungsverfahrens 3 sind Amide wie Hexamethylen-phosphorsäuretriamid, Formamid, *N*-Methyl-formamid, N,N-Dimethyl-formamid, *N,N*-Dipropyl-formamid, *N,N*-Dibutyl-formamid, *N*-Methyl-pyrrolidin, *N*-Methyl-caprolactam, insbesondere N,N-Dimethyl-formamid.

Als basische Reaktionshilfsmittel zur Durchführung des dritten Reaktionsschrittes des erfindungsgemäßen Herstellungsverfahrens 3 werden bevorzugt Hydride oder Carbonate von Alkalimetallen, beispielsweise des Lithiums, Natriums, Kaliums oder Cäsiums eingesetzt.

Zur Durchführung des dritten Reaktionsschrittes des erfindungsgemäßen Herstellungsverfahrens 3 setzt man pro Mol Verbindung der Formel (IX) im allgemeinen 0,5 bis 4,0 Mol, vorzugsweise 0,7 bis 3,0 Mol, besonders bevorzugt 1,0 bis 2,0 Mol an Verbindungen der Formel (X) ein.

Die Reaktionsdauer für den dritten Reaktionsschritt beträgt 5 Minuten bis 48 Stunden. Die Umsetzung erfolgt bei Temperaturen zwischen -10°C und +200°C, bevorzugt zwischen +10°C und 180°C, besonders bevorzugt zwischen 20°C und 140°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre.

Zur Durchführung des ersten Reaktionsschrittes des erfindungsgemäßen Herstellungsverfahrens 3 setzt man pro Mol Verbindung der Formel (II) im allgemeinen 0,5 bis 4,0 Mol, vorzugsweise 0,7 bis 3,0 Mol, besonders bevorzugt 1,0 bis 2,0 Mol an Verbindungen der Formel (VIII) ein.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Ergänzend zu den obigen Angaben zu den Herstellungsverfahren sei auf die Herstellungsbeispiele verwiesen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

Inhibitoren der Nucleinsäure Synthese
Benalaxyl, Benalaxyl-M, Bupirimat, Chiralaxyl, Clozylacon, Dimethirimol, Ethirimol, Furalaxyl, Hymexazol, Mefenoxam, Metalaxyl, Metalaxyl-M, Ofurace, Oxadixyl, Oxolinsäure

Inhibitoren der Mitose und Zellteilung
Benomyl, Carbendazim, Diethofencarb, Ethaboxam, Fuberidazole, Pencycuron, Thiabendazol, Thiophanat-methyl, Zoxamid

Inhibitoren der Atmungskette Komplex I
Diflumetorim

Inhibitoren der Atmungskette Komplex II
Boscalid, Carboxin, Fenfuram, Flutolanil, Furametpyr, Furmecyclox, Mepronil, Oxycarboxin, Penthiopyrad, Thifluzamid

Inhibitoren der Atmungskette Komplex III
Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin

Entkoppler
Dinocap, Fluazinam

Inhibitoren der ATP Produktion
Fentinacetat, Fentinchlorid, Fentinhydroxid, Silthiofarn

Inhibitoren der Aminosäure- und Proteinbiosynthese
Andoprim, Blasticidin-S, Cyprodinil, Kasugamycin, Kasugamycinhydrochlorid Hydrat, Mepanipyrim, Pyrimethanil

Inhibitoren der Signal-Transduktion
Fenpiclonil, Fludioxonil, Quinoxyfen

Inhibitoren der Fett- und Membran Synthese
Chlozolinat, Iprodion, Procymidon, Vinclozolin
Ampropylfos, Kalium-Ampropylfos, Edifenphos, Etridiazol, Iprobenfos (IBP), Isoprothiolan, Pyrazophos
Tolclofos-methyl, Biphenyl
Iodocarb, Propamocarb, Propamocarb hydrochlorid, Propamocarb-Fosetylat

Inhibitoren der Ergosterol Biosynthese
Fenhexamid,
Azaconazol, Bitertanol, Bromuconazol, Cyproconazol, Diclobutrazol, Difenoconazol, Diniconazol, Diniconazol-M, Epoxiconazol, Etaconazol, Fenarimol, Fenbuconazol, Fluquinconazol, Flurprimidol, Flusilazol, Flutriafol, Furconazol, Furconazol-cis, Hexaconazol, Imazalil, Imazalilsulfat Imibenconazol, Ipconazol, Metconazol, Myclobutanil, Nuarimol, Oxpoconazol, Paclobutrazol, Penconazol, Pefurazoat Prochloraz, Propiconazol, Prothioconazol, Pyrifenox, Simeconazol, Tebuconazol, Tetraconazol, Triadimefon, Triadimenol, Triflumizol Triforin, Triticonazol, Uniconazol, Voriconazol, Viniconazol,
Aldimorph, Dodemorph, Dodemorphacetat, Fenpropidin, Fenpropimorph, Spiroxamin, Tridemorph,
Naftifin, Pyributicarb, Terbinafin

Inhibitoren der Zellwand Synthese
Benthiavalicarb, Bialaphos, Dimethomorph, Flumorph, Iprovalicarb, Mandipropamid, Polyoxins, Polyoxorim, Validamycin A

Inhibitoren der Melanin Biosynthese
Capropamid, Diclocymet, Fenoxanil, Phtalid, Pyroquilon, Tricyclazol

Resistenzinduktion
Acibenzolar-S-methyl, Probenazol, Tiadinil

Multisite
Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram

Weitere Fungizide
Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ferimzon, Flumetover, Flusulfamid, Fluopicolid, Fluoroimid, Fosetyl-Aluminium, Fosetyl-Caclcium, Fosetyl-Natrium, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorphenol und Salze, 2-Phenylphenol und Salze, Piperalin, Propanosin-Natrium, Proquinazid, Pyribencarb, Pyrrolnitrin, Quintozen, Tecloftalam, Tecnazen, Triazoxid, Trichlamid, Valiphenal, Zarilamid,
2-(2-{[6-(3-Chlor-2-methylphenoxy)-5-fluorpyrimidin-4-yl]oxy}phenyl)-2-(methoxyimino)-N-methylacetamid,
2-[[[[1-[3(1Fluor-2-phenylethyl)oxy]phenyl]ethyliden]amino]oxy]methyl]-alpha-(methoxyimino)-N-methyl-alphaE-benzacetamid,
cis-1-(4-Chlorphenyl)-2-(1H-1,2,4-triazol-1-yl)-cycloheptanol,
1-[(4-Methoxyphenoxy)methyl]-2,2-dimethylpropyl-1H-imidazol-1- carbonsäure,
2,3,5,6-Teirachlor-4-(methylsulfonyl)-pyridin,
2-Butoxy-6-iod-3-propyl-benzopyranon-4-on,
2-Chlor-N-(2,3-dihydro-1,1,3-trimethyl-1H-inden-4-yl)-3-pyridincarboxamid,
3,4,5-Trichlor-2,6-pyridindicarbonitril,
3,4-Dichlor-N-(2-cyanophenyl)isothiazol-5-carboxamid (Isotianil)
3-[5-(4-Chlorphenyl)-2,3-dimethylisoxazolidin-3-yl]pyridin,
5-Chlor-6-(2,4,6-trifluorophenyl)-N-[(1R)-1,2,2-trimethylpropyl][1,2,4]triazolo[1,5-a]pyrimidin-7-amin,
5-Chlor-7-(4-methylpiperidin-1-yl)-6-(2,4,6-trifluorophenyl)[1,2,4]triawlo[1,5-a]pyrimidin,
5-Chlor-N-[(1R)-1,2-dimethylpropyl]-6-(2,4,6-trifluorophenyl) [1,2,4]triazolo[1,5-a]pyrimidin-7-amin,
Methyl 2-[[[cyclopropyl[(4-methoxyphenyl)imino]methyl]thio]methyl]-.alpha: (methoxymethylen)- benzacetat,
Methyl 1-(2,3-dihydro-2,2-dimethyl-1H-inden-1-yl)-1H-imidazole-5-carboxylat,
N-(3',4'-dichlor-5-fluorbiphenyl-2-yl)-3-(difluormethyl)-1-methyl-1H-pyrazol-4-carboxamid,
N-(3-Ethyl-3,5,5-trimethyl-cyclohexyl)-3-formylamino-2-hydroxy-benzamid,
N-(4-Chlor-2-nitrophenyl)-N-ethyl-4-methyl-benzenesulfonamid,
N-(4-chlorbenzyl)-3-[3-methoxy-4-(prop-2-yn-1-yloxy)phenyl]propanamid,
N-[(4-chlorphenyl)(cyano)methyl]-3-[3-methoxy-4-(prop-2-yn-1-yloxy)phenyl]propanamid,
N-(5-Brom-3-chlorpyridin-2-yl)meihyl-2,4-dichlornicotinamid,
N-[1-(5-Brom-3-chloropyridin-2-yl)ethyl]-2,4-dichloronicotinamid,
(2S)-N-[2-[4-[[3-(4-chlorophenyl)-2-propinyl]oxy]-3-methoxyphenyl]ethyl]- 3-methyl-2-[(methylsulfonyl)amino]-butanamid,
N-{(Z)-[(cyclopropylmethoxy)imino][6-(difluormethoxy)-2,3-difluorphenyl]methyl}-2-benzacetamid,
N-{2-[1,1'-bi(cyclopropyl)-2-yl]phenyl}-3-(difluormethyl)-1-methyl-1H-pyrazol-4-carboxamid,
N-{2-[3-Chlor-5-(trifluormethyl)pyridin-2-yl]ethyl}-2-(trifluoromethyl)benzamid,
N-ethyl-N-methyl-N'-{2-methyl-5-(trifluormethyl)-4-[3-(trimethylsilyl)propoxy]phenyl}imidoformamid,
O-[1-[(4-Methoxyphenoxy)methyl]-2,2-dimethylpropyl]-1H-imidazol-1-carbothioic acid,
2-Amino-4-methyl-N-phenyl-5-thiazolcarboxamid,
2,4-Dihydro-5-methoxy-2-methyl-4-[[[[1-[3-(trifluoromethyl)-phenyl]-ethyliden]-amino]-oxy]-methyl]-phenyl]-3H-1,2,4-triazol-3-on (CAS Nr. 185336-79-2),
N-(6-Methoxy-3-pyridinyl)-cyclopropan carboxamid,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
Carbamate,
zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
Organophosphate,
zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/- ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

Natrium-Kanal-Modulatoren / Spannungsabhängige Natrium-Kanal-Blocker
Pyrethroide,
zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
DDT
Oxadiazine,
zum Beispiel Indoxacarb
Semicarbazon,
zum Beispiel Metaflumizon (BAS3201)

Acetylcholin-Rezeptor-Agonisten/-Antagonisten
Chloronicotinyle,
zum Beispiel Acetamiprid, Clothianidin, Dinotefuran, Imidacloprid, Nitenpyram, Nithiazine, Thiacloprid, Thiamethoxam, AKD-1022, Imidaclothiz
Nicotine, Bensultap, Cartap

Acetylcholin-Rezeptor-Modulatoren
Spinosyne,
zum Beispiel Spinosad und Spinetoram (XDE-175; WO 9700265 A1)

GABA-gesteuerte Chlorid-Kanal-Antagonisten
Organochlorine,
zum Beispiel Camphechlor, Chlordane, Endosulfan, Gamma-HCH, HCH, Heptachlor, Lindane, Methoxychlor
Fiprole,
zum Beispiel Acetoprole, Ethiprole, Fipronil, Pyrafluprole, Pyriprole, Vaniliprole

Chlorid-Kanal-Aktivatoren
Mectine,
zum Beispiel Abamectin, Emamectin, Emamectin-benzoate, Ivermectin, Lepimectin, Milbemycin

Juvenihormon-Mimetika,
zum Beispiel Diofenolan, Epofenonane, Fenoxycarb, Hydroprene, Kinoprene, Methoprene, Pyriproxifen, Triprene

Ecdysonagonisten/disruptoren
Diacylhydrazine,
zum Beispiel Chromafenozide, Halofenozide, Methoxyfenozide, Tebufenozide

Inhibitoren der Chitinbiosynthese
Benzoylharnstoffe,
zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
Buprofezin
Cyromazine

Inhibitoren der oxidativen Phosphorylierung, ATP-Disruptoren
Diafenthiuron
Organozinnverbindungen,
zum Beispiel Azocyclotin, Cyhexatin, Fenbutatin-oxide

Entkoppler der oxidativen Phoshorylierung durch Unterbrechung des H-Protongradienten
Pyrrole,
zum Beispiel Chlorfenapyr
Dinitrophenole,
zum Beispiel Binapacyrl, Dinobuton, Dinocap, DNOC, Meptyldinocap

Seite-I-Elektronentransportinhibitoren
METI's,
zum Beispiel Fenazaquin, Fenpyroximate, Pyrimidifen, Pyridaben, Tebufenpyrad, Tolfenpyrad
Hydramethylnon
Dicofol

Seite-II-Elektronentransportinhibitoren
Rotenone

Seite-III-Elektronentransportinhibitoren
Acequinocyl, Fluacrypyrim

Mikrobielle Disruptoren der Insektendarmmembran
Bacillus thuringiensis-Stämme

Inhibitoren der Fettsynthese
Tetronsäuren,
zum Beispiel Spirodiclofen, Spiromesifen,
Tetramsäuren,
zum Beispiel Spirotetramat, cis-3-(2,5-dimethylphenyl)-4-hydroxy-8-methoxy-1-azaspiro[4.5]dec-3-en-2-on
Carboxamide,
zum Beispiel Flonicamid
Oktopaminerge Agonisten,
zum Beispiel Amitraz

Inhibitoren der Magnesium-stimulierten ATPase,
Propargite
Nereistoxin-Analoge,
zum Beispiel Thiocyclam hydrogen oxalate, Thiosultap-sodium

Agonisten des Ryanodin-Rezeptors,
Benzoesäuredicarboxamide,
zum Beispiel Flubendiamid
Anthranilamide,
zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)

Biologika, Hormone oder Pheromone
Azadirachtin, Bacillus spec., Beauveria spec., Codlemone, Metarrhizium spec., Paecilomyces spec., Thuringiensin, Verticillium spec.

Wirkstoffe mit unbekannten oder nicht spezifischen Wirkmechanismen
Begasungsmittel,
zum Beispiel Aluminium phosphide, Methyl bromide, Sulfuryl fluoride
Fraßhemmer,
zum Beispiel Cryolite, Flonicamid, Pymetrozine
Milbenwachstumsinhibitoren,
zum Beispiel Clofentezine, Etoxazole, Hexythiazox
Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Haltern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Methode 1

### N-[(6-Chlorpyridin-3-yl)methyl]-N'-cyan-N-(2,2-difluorethyl)ethanimidamid

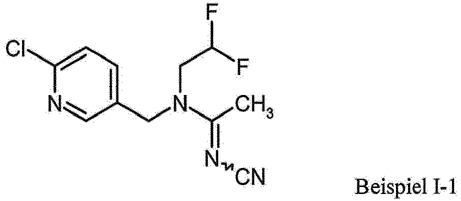

6.5 g (44.2 mmol) N'-Cyan-N-(2,2-difluorethyl)ethanimidamid werden in 250 mL Acetonitril verrührt und nacheinander mit 18.7 g (57.4 mmol) Caesiumcarbonat, 8.6 g (53.0 mmol) 2-Chlor-5-(chlormethyl)pyridin und 1.1 g (4.4 mmol) Caesiumiodid versetzt. Anschließend wird das Reaktionsgemisch 5 Stunden bei Rückflusstemperatur gerührt, anschließend im Vakuum eingeengt und der verbleibende Rückstand mittels Säulenchromatographie über Kieselgel mit dem Laufmittelgemisch Essigsäureethylester : Cyclohexan (3:1) gereinigt. Man erhält 4.7 g (37 % der Theorie) *N*-[(6-Chlorpyridin-3-yl)methyl]-*N*'-cyan-*N*-(2,2-difluorethyl)ethanimidamid.
¹H-NMR (CDCl₃): δ [ppm] = 2.52 + 2.53 (2 s, 3 H), 3.77 (m, 2 H), 4.75 + 4.84 (2 s, 2 H), 5.97 + 6.14 (2 tt, 1 H), 7.35 + 7.42 (2 d, 1 H), 7.48 +7.68 (2 dd, 1 H), 8.26 + 8.30 (2 d, 1 H).
¹³C-NMR (CDCl₃): δ [ppm] = 19.3, 19.4, 49.8, 50.6, 50.8, 51.9, 112.5, 113.0, 115.9, 116.2, 124.7, 125.0, 136.7, 136.8, 137.0, 139.0, 147.9, 149.1, 151.6, 152.1, 173.5, 173.7.
LC-MS: *m*/*z* = 273.1 (M+H⁺, 100%)

Wie man an der Signalverdopplung in den ¹H- und ¹³C-NMR-Spektren erkennen kann, liegt das N-[(6-Chlorpyridin-3-yl)methyl]-N'-cyan-N-(2,2-difluorethyl)ethanimidamid als ein Gemisch aus *E*/*Z*-Isomeren vor.

Analog können die in der nachstehenden Tabelle 1 aufgeführten Verbindungen der Formel (I) hergestellt werden.

**Tabelle 1**

| | | | | | |
|---|---|---|---|---|---|
| 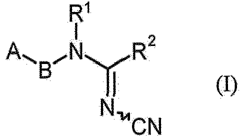 | | | | | |

| **Bsp.-Nr.** | **A** | **B** | **R¹** | **R²** | **Physikalische Daten ^{a)}** |
|---|---|---|---|---|---|
| I-2 | 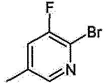 | CH₂ | CH₂CHF₂ | CH₃ | δ = 2.41 + 2.46 (2s, 3H), 3.86 (m, 2H), 4.75 + 4.78 (2s, 2H), 6.06 + 6.13 (2tt, 1H), 7.48 (m, 1H), 8.12 + 8.14 (2s, 1H); LC-MS: *m*/*z* = 335 (⁷⁹Br) und 337 (⁸¹Br) (M+H⁺, 100%). |
| I-3 | 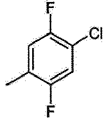 | CH(CH₃) | CH₂CHF₂ | CH₃ | LC-MS: *m*/*z* = 322.0 (M+H⁺, 100%). |
| I-5 | 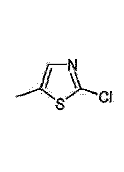 | CH₂ | CH₂CHF₂ | CH₃ | δ=2.39+2.46 (2s, 3H), 3.85 (m, 2H), 4.78 + 4.82 (2s, 2H), 6.05 + 6.11 (2tm, 1H), 7.49 + 7.55 (2s, 1H); LC-MS: *m*/*z* = 278.9 (M+H⁺, 100%). |
| I-6 | 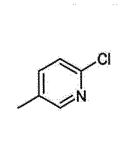 | CH₂ | CH₂CHF₂ | C₂H₅ | δ = 1.23 (m, 3H), 2.78 (m, 2H), 3.84 (m, 2H), 4.76 (s, 2H), 6.09 (m, 1H), 7.37 (m, 1H), 7.61 (m, 1H), 8.26 (s, 1H); LC-MS: *m*/*z* = 287.0 (M+H⁺, 100%). |
| I-7 | 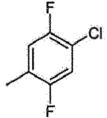 | (CH)CH₃ | CH₂CHF₂ | C₂H₅ | LC-MS: *m*/*z* = 336.0 (M+H⁺, 100%). |
| I-8 | 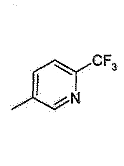 | CH₂ | CH₂CHF₂ | CH₃ | δ = 2.41 + 2.47 (2s, 3H), 3.89 (m, 2H), 4.85 + 4.87 (2s, 2H), 6.07 + 6.15 (2tt, 1H), 7.78 (m, 2H), 8.61 (s, 1H); LC-MS: *m*/*z* = 307.0 (M+H⁺, 100%). |
| I-9 | 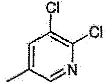 | CH₂ | CH₂CHF₂ | CH₃ | δ = 2.41 + 2.45 (2s, 3H), 3.86 (m, 2H), 4.74 + 4.77 (2s, 2H), 6.06 + 6.13 (2tt, 1H), 7.78 + 7.82 (2s, 1H), 8.20 + 8.23 (2s, 1H); LC-MS: *m*/*z* = 307.0 (M+H⁺, 100%). |
| I-10 | 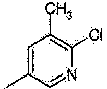 | CH₂ | CH₂CHF₂ | CH₃ | δ = 2.35 + 2.36 + 2.42 + 2.46 (4s, 6H), 3.84 (m, 2H), 4.70 + 4.75 (2s, 2H), 6.04 + 6.12 (2tt, 1H), 7.51 + 7.56 (2s, 1H), 8.08 + 8.11 (2s, 1H); LC-MS: *m*/*z* = 287.0 (M+H⁺, 100%). |

| | | | | | |
|---|---|---|---|---|---|
| ^{a) 1}H-NMR (CD₃CN) | | | | | |

**Die folgenden Beispiele I-17, I-18 und 1-19 werden nicht vom Schutzumfang der Patentansprüche umfasst.**

### Methode 2

### N-[(6-Chlorpyridin-3-yl)methyl]-N'-cyano-N-(3-fluorpropyl)ethanimidamid

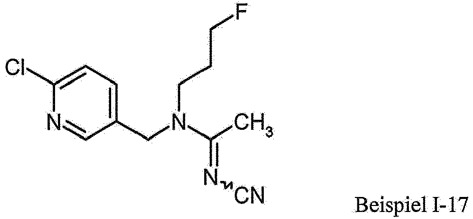

500 mg (2.47 mmol) *N*-[(6-Chlorpyridin-3-yl)methyl]-3-fluorpropan-1-amin und 242 mg (2.47 mmol) *N*-Cyanethanimidoessigsäuremethylester werden in 10 mL Methanol 4 Tage bei Raumtemperatur gerührt. Nach dem Einengen des Reaktionsansatzes im Vakuum nimmt man mit Essigsäureethylester auf, wäscht nacheinander zweimal mit 1 N wässriger Salzsäure, zweimal mit 1 N wässriger Natriumhydroxid-Lösung und gesättigter Natriumchlorid-Lösung und trocknet über Natriumsulfat. Nach Einengen der organischen Phase im Vakuum und Reinigung des Rückstandes durch Säulenchromatographie über Kieselgel mit dem Laufmittelgemisch Essigsäureethylester : Cyclohexan (1:1) erhält man 56 mg (8 % der Theorie) *N*-[(6-Chlorpyridin-3-yl)methyl]-*N*'-cyano-N-(3-fluorpropyl)ethanimidamid.
¹H-NMR (CD₃CN): δ [ppm] = 2.38 + 2.43 (2 s, 3 H), 3.54 (m, 2 H), 4.47 (dm, 2 H), 4.65 + 4.70 (2 s, 2 H), 7.38 (m, 1 H), 7.65 (m, 1 H), 8.29 (m, 1 H).
LC-MS: *m*/*z* = 269.1 (M+H⁺, 100%)

In analoger Weise kann dargestellt werden:
***N*-[(6-Chlorpyridin-3-yl)methyl]-*N*'-cyan-N-(2,2-difluorethyl)ethanimidamid** (Analytik vgl. Beispiel I-1).

### Methode 3

### N-[(6-Chlorpyridin-3-yl)methyl]-N'-cyano-N-(3,3-dichlorprop-2-en-1-yl)ethanimidamid

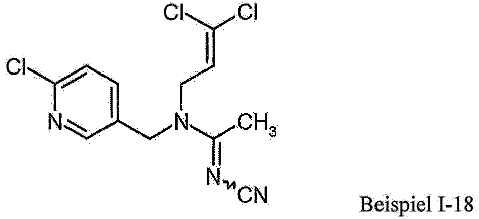

174 mg (0.83 mmol) *N*-[(6-Chlorpyridin-3-yl)methyl]-*N*'-cyanoethanimidamid werden in 5 mL Acetonitril verrührt und nacheinander mit 543 mg (1.67 mmol) Caesiumcarbonat, 166 mg (0.88 mmol) 3-Brom-1,1-dichlorprop-1-en und 22 mg (0.08 mmol) Caesiumiodid versetzt. Anschließend wird das Reaktionsgemisch 1 Stunde bei Rückflusstemperatur gerührt. Nach dem Einengen des Reaktionsansatzes im Vakuum nimmt man mit Essigsäureethylester auf, wäscht nacheinander mit 1 N wässriger Salzsäure, 1 N wässriger Natriumhydroxid-Lösung und gesättigter Natriumchlorid-Lösung und trocknet über Natriumsulfat. Nach Einengen der organischen Phase im Vakuum und Reinigung des Rückstandes durch Säulenchromatographie über Kieselgel mit dem Laufmittelgemisch Essigsäureethylester : Cyclohexan (2:1) erhält man 215 mg (77 % der Theorie) *N*-[(6-Chlorpyridin-3-yl)methyl]-*N*'-cyano-*N*-(3,3-dichlorprop-2-en-1-yl)ethanimidamid.
¹H-NMR (CD₃CN): δ [ppm] = 2.43 (s, 3 H), 4.15 (m, 2 H), 4.65 + 4.69 (2 s, 2 H), 6.00 (t, 1 H), 7.39 (m, 1 H), 7.65 (m, 1 H), 8.30 (m, 1 H).
LC-MS: *m*/*z* = 317.0 (M+H⁺, 100%)

In analoger Weise kann dargestellt werden:
***N*-[(6-Chlorpyridin-3-yl)methyl]-*N*'-cyan-N-(2,2-difluorethyl)ethanimidamid** (Analytik vgl. Beispiel I-1).

### N-[(6-Chlorpyridin-3-yl)methyl]-N'-cyano-N-(3,3-difluorprop-2-en-1-yl)ethanimidamid

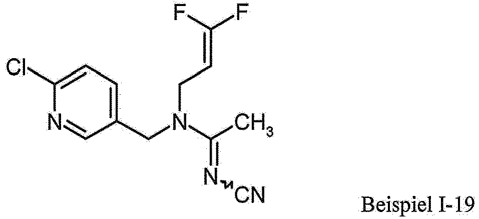

¹H-NMR (CD₃CN): δ [ppm] = 2.40 + 2.44 (2 s, 3 H), 4.03 (m, 2 H), 4.50 (m, 1 H), 4.63 + 4.68 (2 s, 2 H), 7.39 (m, 1 H), 7.65 (m, 1 H), 8.28 (m, 1 H).

LC-MS: *m*/*z* = 285.1 (M+H⁺, 100%)

### Herstellung der Ausgangsverbindungen

### Verbindungen der Formel (HN(R¹)-C(R²)=N-CN) (IV)

### IV-1

### N'-Cyan-N-(2,2-difluorethyl)ethanimidamid

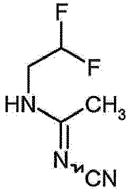

8.3 g (101.9 mmol) 2,2-Difluorethanamin und 10.0 g (101.9 mmol) *N*-Cyanethanimidoessigsäure-methylester werden in 100 mL Methanol 3 Stunden bei Raumtemperatur gerührt. Nach dem Einengen des Reaktionsansatzes im Vakuum erhält man 14.7 g (98% der Theorie) *N*'-Cyan-*N*-(2,2-difluorethyl)ethan imidamid.

¹H-NMR (CD₃CN): δ [ppm] = 2.27 (s, 3 H), 3.67 (m, 2 H), 5.97 (tt, 1 H), 7.03 (br. s, 1 H).

LC-MS: *m*/*z* = 148.1 (M+H⁺, 100%).

Analog kann die in der nachstehenden Tabelle 2 aufgeführte Verbindung der Formel (IV) hergestellt werden.

**Tabelle 2**

| | | | |
|---|---|---|---|
| 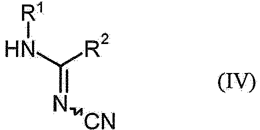 | | | |

| **Bsp.-Nr.** | **R¹** | **R²** | **Physikalische Daten ^{a)}** |
|---|---|---|---|
| IV-3 | CH₂CHF₂ | C₂H₅ | δ = 1.24 (t, 3H), 2.59 (q, 2H), 3.66 (m, 2H), 5.97 (tt, 1H), 6.96 (br. s, 1H); LC-MS: *m*/*z* = 162.1 (M+H⁺, 100%). |

| | | | |
|---|---|---|---|
| ^{a) 1}H-NMR (CD₃CN) | | | |

### Verbindungen der Formel (A-CH₂-E) (V, B = CH₂)

### Va-1

### (5,6-Dichlorpyridin-3-yl)methanol (E = OH, A = 5,6-Dichlor-pyrid-3-yl) (R. Graf et al. J. Prakt. Chem. 1932, 134 177-87)

Zu 110 g (573 mmol) 5,6-Dichlor-nicotinsäure in 250 mL Tetrahydrofuran tropft man bei 0°C 859 mL (859 mmol) einer 1 M Lösung von Boran-Tetrahydrofuran-Komplex in Tetrahydrofuran hinzu. Man erwärmt auf Raumtemperatur und rührt bei dieser Temperatur 3 Stunden. Nach Abkühlen auf 0°C stellt man die Reaktionsmischung mit gesättigter wässriger Kaliumcarbonat-Lösung alkalisch, rotiert das Tetrahydrofuran weitgehend ab und extrahiert den Rückstand mehrmals mit Essigsäureethylester. Die vereinigten organischen Phasen werden mit Wasser und gesättigter wässriger Natriumchlorid-Lösung gewaschen und über Natriumsulfat getrocknet. Nach Einengen der organischen Phase im Vakuum und Reinigung des Rückstandes durch Säulenchromatographie über Kieselgel (Kieselgel 60 - Merck, Korngröße: 0.04 bis 0.063 mm) mit dem Laufmittelgemisch Essigsäureethylester : Cyclohexan (1:2) erhält man 62 g (61 % derTheorie) (5,6-Dichlorpyridin-3-yl)methanol.
¹H-NMR (CD₃CN): δ [ppm] = 3.31 (t, 1 H), 4.60 (d, 2 H), 7.85 (s, 1 H), 8.26 (s, 1 H)

Analog zur Vorschrift der Verbindung (Va-1) wurde auch die Verbindung (Va-5) aus Tabelle 3 hergestellt.

### Va-2

### 3-Brommethyl-5,6-dichlorpyridin (E = Br, A = 5,6-Dichlor-pyrid-3-yl) (vgl. WO 2000046196 A1)

Eine Lösung von 10.60 g (59.55 mmol) (5,6-Dichlorpyridin-3-yl)methanol (Va-1) in 100 mL Dichlormethan versetzt man bei 0°C mit 16.40 g (62.52 mmol) Triphenylphosphin und 11.66 g (65.50 mmol) *N*-Bromsuccinimid. Nach 2 h wird die Reaktionsmischung weitgehend eingeengt und der Rückstand durch Säulenchromatographie über Kieselgel (Kieselgel 60 - Merck, Korngröße: 0.04 bis 0.063 mm) mit dem Laufmittelgemisch Essigsäureethylester : Cyclohexan (1:5) gereinigt. Man erhält 12.4 g (86 % der Theorie) 3-Brommethyl-5,6-dichlorpyridin.

¹H-NMR (CD₃CN): δ [ppm] = 4.53 (s, 2 H), 7.97 (s, 1 H), 8.35 (s, 1 H)

Analog zur Vorschrift der Verbindung (Va-2) wurden die Verbindungen (Va-6) bis (Va-8) aus (Tabelle 3) hergestellt.

### Va-4

### 6-Chlor-3-chlormethyl-5-fluorpyridin (E = Cl, A = 6-Chlor-5-fluor-pyrid-3-yl)

1.00 g (6.87 mmol) 6-Chlor-5-fluor-3-methylpyridin (F. L. Setliff, Organic Preparations and Procedures International 1971, 3, 217-222), 1.01 g (7.56 mmol) *N*-Chlorsuccinimid und 0.11 g (0.69 mmol) 2,2'-Azobis(2-methylpropannitril) in 100 mL Chlorbenzol werden 2 Tage unter Rückfluss gekocht. Dabei werden nach ca. 16 und 32 Stunden jeweils weitere 1.01 g (7.56 mmol) *N*-Chlorsuccinimid und 0.11 g (0.69 mmol) 2,2'-Azobis(2-methylpropannitril) hinzugefügt. Nach Waschen der Reaktionsmischung mit gesättigter wässriger Natriumsulfit-Lösung und Natriumhydrogencarbonat-Lösung trocknet man über Natriumsulfat und engt im Vakuum ein. Säulenchromatographie des Rückstandes über Kieselgel (Kieselgel 60 - Merck, Korngröße: 0.04 bis 0.063 mm) mit dem Laufmittelgemisch Essigsäureethylester : Cyclohexan (1:20) liefert 0.65 g (53 % der Theorie) 6-Chlor-3-chlormethyl-5-fluorpyridin.

¹H-NMR (CD₃CN): δ [ppm] = 4.68 (s, 2 H), 7.69 (d, 1 H), 8.27 (s, 1 H)

In der nachstehenden Tabelle 3 sind weitere Verbindungen (Va-5) bis (Va-10) der Formel (Va) aufgeführt.

**Tabelle 3**

| E-CH₂-A (Va) | | | |
|---|---|---|---|
| Bsp.-Nr. | E | A | Physikalische Daten ^{a)} |
| Va-5 | OH | 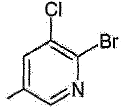 | 3.30 (t, 1H), 4.59 (d, 2H), 7.83 (s, 1H), 8.26 (s, 1H) |
| Va-6 | Br | 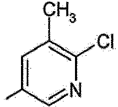 | 2.37 (s, 3H), 4.52 (s, 2H), 7.70 (s, 1H), 8.24 (s, 1H) |
| Va-7 | Br | 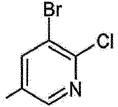 | 4.52 (s, 2H), 8.10 (s, 1H), 8.38 (s, 1H) |
| Va-8 | Br | 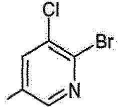 | 4.52 (d, 2H), 7.92 (s, 1H), 8.35 (s, 1H) |
| Va-9 | Br | 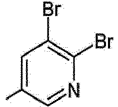 | 4.50 (s, 2 H), 8.07 (s, 1 H), 8.37 (s, 1 H) |
| Va-10 | Br | 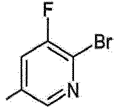 | 4.55 (s, 2H), 7.65 (d, 1H), 8.27 (s, 1H) |

| | | | |
|---|---|---|---|
| ^{a) 1}H-NMR (CD₃CN), δ [ppm] | | | |

### Verbindungen der Formel (A-B-N(R¹)H) (VI)

### VI-1

### N-[(6-chlor-5-fluorpyridin-3-yl)methyl]-2,2-difluorethylamin

520 mg (2.89 mmol) 6-Chlor-3-chlormethyl-5-fluorpyridin (Va-4), 1.05 mL (14.44 mmol) 2,2-Difluorethylamin und 400 µL (2.89 mmol) Triethylamin werden in 50 mL Acetonitril ca. 48 Stunden bei 45°C gerührt. Dabei werden nach ca. 16 und 32 Stunden jeweils weitere 0.42 mL (5.78 mmol) 2,2-Difluorethylamin hinzugefügt. Nach Einengen der Reaktionsmischung im Vakuum nimmt man mit 1 N wässriger Salzsäure auf und wäscht mit Essigsäureethylester. Die wässrige Phase wird mit 2.5 N wässriger Natriumhydroxid-Lösung alkalisch gestellt und mehrmals mit Essigsäureethylester extrahiert. Trocknen der vereinigten organischen Phasen über Natriumsulfat und Einengen im Vakuum liefern 370 mg (57 % der Theorie) N-[(6-chlor-5-fluorpyridin-3-yl)methyl]-2,2-difluorethylamin.
¹H-NMR (CD₃CN): δ [ppm] = 2.95 (td, 2 H), 3.87 (s, 2 H), 5.87 (tt, 1 H), 7.62 (d, 1 H), 8.17 (s, 1 H).

### In analoger Weise kann dargestellt werden:

### VI-2

### N-[(6-Chlorpyridin-3-yl)methyl]-2,2-difluorethan-1-amin

¹H-NMR (CD₃CN, δ, ppm) = 2.93 (td, 2 H), 3.80 (s, 2 H), 5.85 (tt, 1 H), 7.33 (d, 1 H), 7.71 (dd, 1H), 8.30 (d, 1 H).

### Verbindungen der Formel (A-B-NH-C(R²)=N-CN) (IX)

### IX-1

### N-[(6-Chlorpyridin-3-yl)methyl]-N'-cyanoethanimidamid (vgl. WO 9104965 A1)

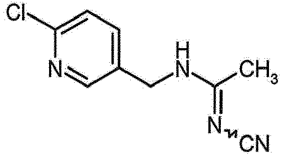

500 mg (3.51 mmol) 1-(6-Chlorpyridin-3-yl)methanamin und 378 mg (3.86 mmol) *N-*Cyanethanimidoessigsäuremethylester werden in 10 mL Methanol 3 Stunden bei Raumtemperatur gerührt. Nach Einengen des Reaktionsansatzes im Vakuum und Reinigung des Rückstandes durch Umkristallisation aus Essigsäureethylester/Cyclohexan erhält man 532 mg (52 % der Theorie) *N-*[(6-Chlorpyridin-3-yl)methyl]-*N*'-cyanoethanimidamid

LC-MS: *m*/*z* = 209.0 (M+H⁺, 100%).

### Biologische Beispiele

### Beispiel Nr. 1

### Myzus-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. I-2, I-5, I-6, I-8, I-9, I-10, I-11, I-12, I-14, I-15, I-16, I-17, I-19, I-18

### Beispiel Nr. 2

### Meloidogyne-Test (Spritzbehandlung)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 20 ppm:
Bsp. Nr. I-3

### Beispiel Nr. 3

### Phaedon-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. I-2, I-9, I-10, I-19

### Beispiel Nr. 4

### Bemisia tabaci (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblattscheiben *(Gossypium hirsutum),* die von Larven der Weißen Fliege *(Bemisia tabaci)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Weiße Fliegen abgetötet wurden; 0 % bedeutet, dass keine Weiße Fliege abgetötet wurde.

Bei diesem Test zeigen z. B. die folgenden Verbindungen der Herstellungsbeispiele eine Wirkung von ≥ 80 % bei einer Aufwandmenge von 500 g/ha: Bsp. Nr. I-2

### Beispiel Nr. 5

### Lucilia cuprina-Test

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. 1-2

### Vergleichsbeispiele

### Beispiel Nr. 1

### Phaedon-Test (Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt)

Nach der gewünschten Zeit wird die Wirkung in % bestimmt. Dabei bedeutet 100 % , dass alle Käferlarven abgetötet wurden; 0 % bedeutet, dass keine Käferlarven abgetötet wurden.

Die Ergebnisse sind in der Tabelle weiter unten wiedergegeben.

### Beispiel Nr. 2

### Ctenocephalides felis; oral

| | |
|---|---|
| Lösungsmittel: | Dimethylsulfoxid |

Zwecks Herstellung einer zweckmäßigen Wirkstoffzubereitung vermischt man 1 Gewichtsteil Wirkstoff mit der angegebenen Menge Wasser. Ein Teil des Konzentrats wird mit citiriertem Rinderblut verdünnt und die gewünschte Konzentration hergestellt.

20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Während das Blut auf 37° C erwärmt wird, ist im Bereich der Flohkammern Raumtemperatur.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Die Ergebnisse sind in der Tabelle weiter unten wiedergegeben.

### Beispiel Nr. 3

### Musca domestica-Test

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica Adulten* besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden.

Die Ergebnisse sind in der folgenden Tabelle wiedergegeben (d = Tage).

| **Wirkstoff** | **Bsp. Nr.** | **1. Phaedon Test Spritzbeh.** | | **2. Ctenocephalides felis** | | **3. Musca domestica** | |
|---|---|---|---|---|---|---|---|
| | | **g/ha** | **% 7 d** | **ppm** | **% 2 d** | **ppm** | **% 2 d** |
| | I-1 | 20 | 100 | 100 | 70 | 100 | 100 |
| | I-6 | | | | | 100 | 50 |
| 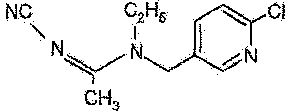 | | 20 | 0 | 100 | 30 | 100 | 0 |
| Bekannt aus | | | | | | | |
| WO 91/04965 | | | | | | | |

## Patentansprüche

1. Verbindungen der Formel (I) 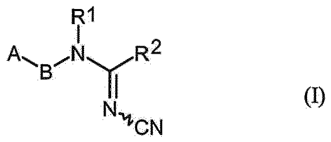 in welcher
A für 6-Chlor-pyrid-3-yl, 6-Brom-pyrid-3-yl, 6-Methyl-pyrid-3-yl, 6-Trifluormethyl-pyrid-3-yl, 2-Methyl-pyrimidin-5-yl, 2-Chlor-pyrimid-5-yl, für 1*H*-Pyrazol-4-yl, welches gegebenenfalls in 1-Position substituiert ist durch Methyl oder Ethyl, und in 3-Position durch Chlor, für 1*H*-Pyrazol-5-yl, 3-Methyl-pyrazol-5-yl, 2-Brom-1,3-thiazol-5-yl, 2-Chlor-1,3-thiazol-5-yl, für Isoxazol-5-yl, welches gegebenenfalls in 3-Position substituiert ist durch Methyl, Ethyl, Chlor oder Brom, 3-Methyl-1,2,4-oxadiazol-5-yl, 1-Methyl-1,2,4-triazol-3-yl oder 1,2,5-Thiadiazol-3-yl, steht,
oder worin
A für 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl oder 5-Methyl-6-brom-pyrid-3-yl, steht,
oder worin
A für einen Rest 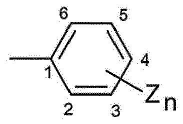
steht,
in welchem
n für 2 oder 3 steht und
Z für Fluor oder Chlor steht,
R¹ für 2,2-Difluorethyl steht,
R² für Methyl oder Ethyl steht und
B für einen Rest aus der Reihe (B-1) bis (B-9) 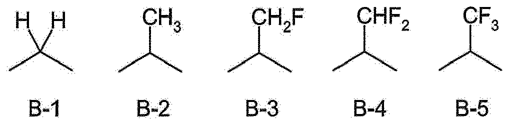 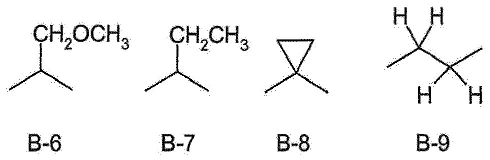
steht.

2. Verbindungen der Formel (I) gemäß Anspruch 1,
worin
A für einen Rest aus der Reihe 6-Chlor-pyrid-3-yl, 6-Brom-pyrid-3-yl, 2-Methyl-pyrimidin-5-yl, 2-Chlor-pyrimid-5-yl, 3-Methyl-isoxazol-5-yl, 3-Brom-isoxazol-5-yl, 5-Fluor-6-chlor-pyrid-3-yl, 5,6-Dichlor-pyrid-3-yl, 5-Fluor-6-brom-pyrid-3-yl, 2-Chlor-1,3,-thiazol-5-yl, steht,
R¹ für 2,2-Difluorethyl steht,
R² für Methyl steht und
B für Methylen (-CH₂-) steht.

3. Verfahren zur Herstellung von Verbindungen der Formel (I) gemäß Anspruch 1, **dadurch gekennzeichnet, dass**
a) gemäß der Herstellungsmethode 1 Verbindungen der Formel (II) 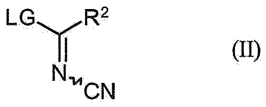 in welcher
R² die oben genannte Bedeutung hat und LG für eine geeignete Abgangsgruppe (leaving group), beispielsweise C₁₋₂-alkoxy steht,
in einem ersten Reaktionsschritt mit Verbindungen der Formel (III)
R¹-NH₂ (III)
in welcher
R¹ die oben genannte Bedeutung hat,
gegebenenfalls in Gegenwart eines geeigneten Verdünnungsmittels und gegebenenfalls in Gegenwart eines basischen Hilfsmittels zu Verbindungen der Formel (IV) umsetzt, 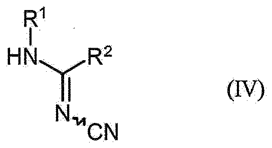 in welcher
R¹ und R² die oben genannte Bedeutung haben,
und diese dann in einem zweiten Reaktionsschritt mit Verbindungen der Formel (V)
A-B-E (V)
in welcher
A und B die oben genannte Bedeutung haben,
E für eine geeignete Abgangsgruppe (leaving group) LG steht,
gegebenenfalls in Gegenwart eines geeigneten Verdünnungsmittels und gegebenenfalls in Gegenwart eines basischen Hilfsmittels umsetzt, oder indem man
b) gemäß der Herstellungsmethode 2 Verbindungen der Formel (II) 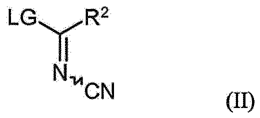 in welcher
R² und LG die oben genannten Bedeutungen haben
mit Verbindungen der Formel (VI)
A-B-N(R¹)H (VI)
in welcher
A, B und R¹ die oben genannte Bedeutung haben,
gegebenenfalls in Gegenwart eines geeigneten Verdünnungsmittels und gegebenenfalls in Gegenwart eines basischen Hilfsmittels umsetzt, oder indem man
c) gemäß der Herstellungsmethode 3 Orthoester der Formel (VII) 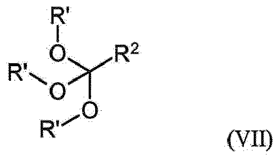 in welcher
R² die oben genannte Bedeutung hat und
R' für Methyl oder Ethyl steht
in einem ersten Reaktionsschritt mit Cyanamid gegebenenfalls in Gegenwart von Verdünnungsmitteln *in-situ* zu Verbindungen der Formel (II) umsetzt 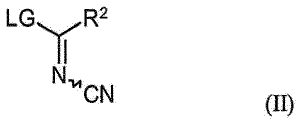 in welcher
R² und LG die oben genannten Bedeutungen haben,
und diese dann in einem zweiten Reaktionsschritt mit Verbindungen der Formel (VIII)
A-B-NH₂ (VIII)
in welcher
A, und B die oben genannten Bedeutungen haben,
gegebenenfalls in Gegenwart eines geeigneten Verdünnungsmittels und gegebenenfalls in Gegenwart eines basischen Hilfsmittels zu Verbindungen der Formel (IX) umsetzt, 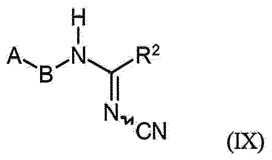 in welcher
A, B, R² die oben genannte Bedeutungen haben,
und diese dann in einem dritten Reaktionsschritt mit Verbindungen der Formel (X)
R¹-E (X)
in welcher
R¹ und E die oben genannten Bedeutungen haben,
gegebenenfalls in Gegenwart eines geeigneten Verdünnungsmittels und gegebenenfalls in Gegenwart eines basischen Hilfsmittels umsetzt.

4. Verbindung der Formel (I) gemäß Anspruch 1, wobei die Verbindung der Formel (I) die Verbindung der Formel (I-1) 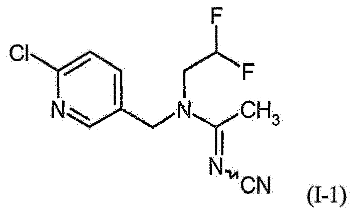 ist.

5. Verbindung der Formel (IV-1)

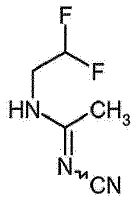

6. Mittel, **gekennzeichnet durch** einen Gehalt von mindestens einer Verbindung der Formel (I) gemäß Anspruch 1 und üblichen Streckmitteln und/oder oberflächenaktiven Substanzen.

7. Nichttherapeutische Verfahren zum Bekämpfen von Schädlingen **dadurch gekennzeichnet, dass** man eine Verbindung der Formel (I) gemäß Anspruch 1 oder ein Mittel gemäß Anspruch 6 auf die Schädlinge und/oder ihren Lebensraum einwirken lässt.

8. Verwendung von Verbindungen der Formel (I) gemäß Anspruch 1 oder von Mitteln gemäß Anspruch 6 zum nichttherapeutischen Bekämpfen von Schädlingen.

## Claims

1. Compounds of the formula (I) 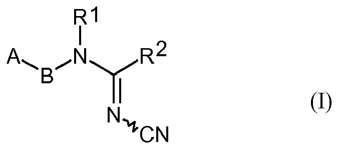 in which
A represents 6-chloropyrid-3-yl, 6-bromopyrid-3-yl, 6-methylpyrid-3-yl, 6-trifluoromethyl-pyrid-3-yl, 2-methylpyrimidin-5-yl, 2-chloropyrimid-5-yl, represents 1*H*-pyrazol-4-yl which is optionally substituted in the 1-position by methyl or ethyl and in the 3-position by chlorine, represents 1*H*-pyrazol-5-yl, 3-methylpyrazol-5-yl, 2-bromo-1,3-thiazol-5-yl, 2-chloro-1,3-thiazol-5-yl, represents isoxazol-5-yl which is optionally substituted in the 3-position by methyl, ethyl, chlorine or bromine, 3-methyl-1,2,4-oxadiazol-5-yl, 1-methyl-1,2,4-triazol-3-yl or 1,2,5-thiadiazol-3-yl,
or in which
A represents 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5,6-dibromopyrid-3-yl, 5-methyl-6-chloropyrid-3-yl or 5-methyl-6-bromopyrid-3-yl,
or in which
A represents a radical 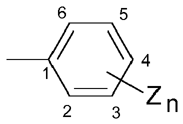
in which
n represents 2 or 3 and
Z represents fluorine or chlorine,
R¹ represents 2,2-difluoroethyl,
R² represents methyl or ethyl and
B represents a radical from the group consisting of (B-1) to (B-9)

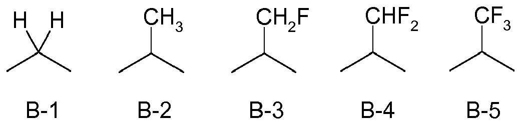

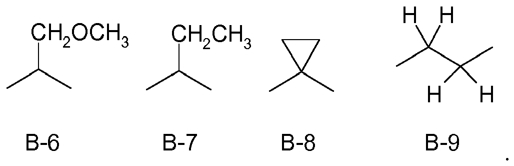

2. Compounds of the formula (I) according to Claim 1
in which
A represents a radical from the group consisting of 6-chloropyrid-3-yl, 6-bromopyrid-3-yl, 2-methylpyrimidin-5-yl, 2-chloropyrimid-5-yl, 3-methylisoxazol-5-yl, 3-bromoisoxazol-5-yl, 5-fluoro-6-chloropyrid-3-yl, 5,6-dichloropyrid-3-yl, 5-fluoro-6-bromopyrid-3-yl, 2-chloro-1,3-thiazol-5-yl,
R¹ represents 2,2-difluoroethyl,
R² represents methyl and
B represents methylene (-CH₂-).

3. Process for preparing compounds of the formula (I) according to Claim 1, **characterized in that**
a) according to preparation method 1, compounds of the formula (II) 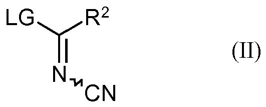 in which
R² is as defined above and LG represents a suitable leaving group, for example C₁-C₂-alkoxy are,
in a first reaction step, reacted with compounds of the formula (III)
R¹-NH₂ (III)
in which
R¹ is as defined above,
if appropriate in the presence of a suitable diluent and if appropriate in the presence of a basic auxiliary, to give compounds of the formula (IV) 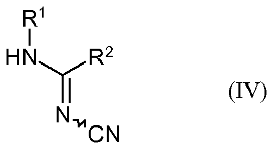 in which
R¹ and R² are as defined above,
and these are then, in a second reaction step, reacted with compounds of the formula (V)
A-B-E (V)
in which
A and B are as defined above,
E represents a suitable leaving group LG,
if appropriate in the presence of a suitable diluent and if appropriate in the presence of a basic auxiliary or
b) according to preparation method 2, compounds of the formula (II) 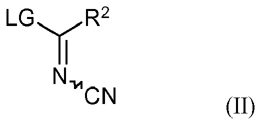 in which
R² and LG are as defined above
are reacted with compounds of the formula (VI)
A-B-N(R¹)H (VI)
in which
A, B and R¹ are as defined above,
if appropriate in the presence of a suitable diluent and if appropriate in the presence of a basic auxiliary, or
c) according to preparation method 3, orthoesters of the formula (VII) 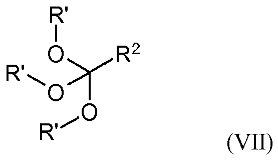 in which
R² is as defined above and
R' represents methyl or ethyl
are, in a first reaction step, reacted in situ with cyanamide, if appropriate in the presence of diluents, to give compounds of the formula (II) 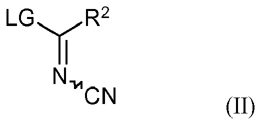 in which
R² and LG are as defined above, and these are then, in a second reaction step, reacted with compounds of the formula (VIII)
A-B-NH₂ (VIII)
in which
A and B are as defined above,
if appropriate in the presence of a suitable diluent and if appropriate in the presence of a basic auxiliary, to give compounds of the formula (IX) 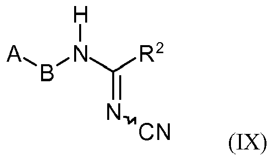 in which
A, B, R² are as defined above,
and these are then, in a third reaction step, reacted with compounds of the formula (X)
R¹-E (X)
in which
R¹ and E are as defined above,
if appropriate in the presence of a suitable diluent and if appropriate in the presence of a basic auxiliary.

4. Compound of the formula (I) according to Claim 1, wherein the compound of the formula (I) is the compound of the formula (I-1)

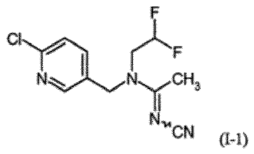

5. Compound of the formula (IV-1)

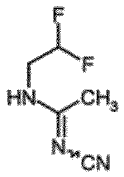

6. Compositions, **characterized in that** they comprise at least one compound of the formula (I) according to Claim 1 and customary extenders and/or surfactants.

7. Non-therapeutic method for controlling pests, **characterized in that** a compound of the formula (I) according to Claim 1 or a composition according to Claim 6 is allowed to act on the pests and/or their habitat.

8. Use of compounds of the formula (I) according to Claim 1 or of compositions according to Claim 6 for the non-therapeutic control of pests.

## Revendications

1. Composés de formule (I) 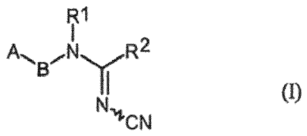 dans laquelle
A représente 6-chloro-pyrid-3-yle, 6-bromo-pyrid-3-yle, 6-méthyl-pyrid-3-yle, 6-trifluorométhyl-pyrid-3-yle, 2-méthyl-pyrimidin-5-yle, 2-chloro-pyrimid-5-yle, 1H-pyrazol-4-yle, qui est éventuellement substitué en position 1 par méthyle ou éthyle, et en position 3 par chlore, 1H-pyrazol-5-yle, 3-méthyl-pyrazol-5-yle, 2-bromo-1,3-thiazol-5-yle, 2-chloro-1,3-thiazol-5-yle, isoxazol-5-yle, qui est éventuellement substitué en position 3 par méthyle, éthyle, chlore ou brome, 3-méthyl-1,2,4-oxadiazol-5-yle, 1-méthyl-1,2,4-triazol-3-yle ou 1,2,5-thiadiazol-3-yle,
ou dans laquelle
A représente 5-fluoro-6-chloro-pyrid-3-yle, 5,6-dichloro-pyrid-3-yle, 5-bromo-6-chloro-pyrid-3-yle, 5-fluor-6-bromo-pyrid-3-yle, 5-chloro-6-bromo-pyrid-3-yle, 5,6-dibromo-pyrid-3-yle, 5-méthyl-6-chloro-pyrid-3-yle ou 5-méthyl-6-bromo-pyrid-3-yle, ou dans laquelle
A représente un radical 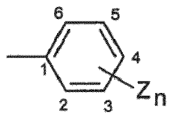
dans lequel
n représente 2 ou 3, et
Z représente fluor ou chlore,
R¹ représente 2,2-difloroéthyle,
R² représente méthyle ou éthyle, et
B représente un radical de la série (B-1) à (B-9)

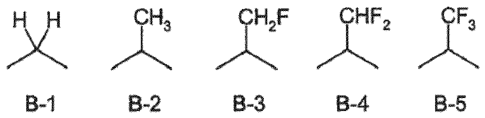

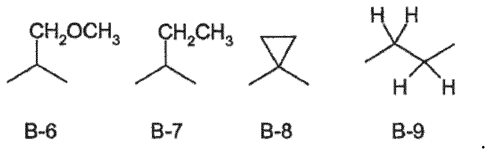

2. Composés de formule (I) selon la revendication 1, dans lesquels
A représente un radical de la série 6-chloro-pyrid-3-yle, 6-bromo-pyrid-3-yle, 2-méthyl-pyrimidin-5-yle, 2-chloro-pyrimid-5-yle, 3-méthyl-isoxazol-5-yle, 3-bromo-isoxazol-5-yle, 5-fluoro-6-chloro-pyrid-3-yle, 5,6-dichloro-pyrid-3-yle, 5-fluoro-6-bromo-pyrid-3-yle, 2-chloro-1,3-thiazol-5-yle,
R¹ représente 2,2-difluoroéthyle,
R² représente méthyle, et
B représente méthylène (-CH₂-).

3. Procédé de fabrication de composés de formule (I) selon la revendication 1, **caractérisé en ce que**
a) selon la méthode de fabrication 1, des composés de formule (II) 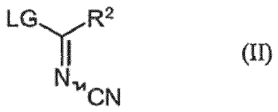 dans laquelle
R² a la signification indiquée précédemment et LG représente un groupe partant (leaving group), par exemple alcoxy en C₁₋₂,
sont mis en réaction lors d'une première étape de réaction avec des composés de formule (III)
R¹-NH₂ (III)
dans laquelle
R¹ a la signification indiquée précédemment,
éventuellement en présence d'un diluant approprié et éventuellement en présence d'un adjuvant basique, pour former des composés de formule (IV) 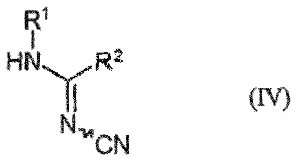 dans laquelle
R¹ et R² ont la signification indiquée précédemment,
puis ceux-ci sont mis en réaction lors d'une seconde étape de réaction avec des composés de formule (V)
A-B-E (V)
dans laquelle
A et B ont la signification indiquée précédemment,
E représente un groupe partant LG (leaving group) approprié,
éventuellement en présence d'un diluant approprié et éventuellement en présence d'un adjuvant basique, ou **en ce que**
b) selon la méthode de fabrication 2, des composés de formule (II) 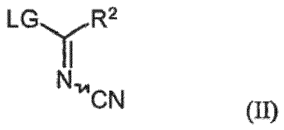 dans laquelle
R² et LG ont les significations indiquées précédemment, sont mis en réaction avec des composés de formule (VI)
A-B-N(R¹)H (VI)
dans laquelle
A, B et R¹ ont la signification indiquée précédemment,
éventuellement en présence d'un diluant approprié et éventuellement en présence d'un adjuvant basique, ou **en ce que**
c) selon la méthode de fabrication 3, des orthoesters de formule (VII) 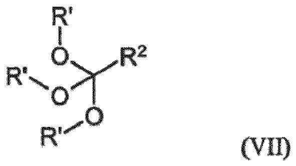 dans laquelle
R² a la signification indiquée précédemment et
R' représente méthyle ou éthyle,
sont mis en réaction lors d'une première étape de réaction avec du cyanamide, éventuellement en présence de diluants, pour former in situ des composés de formule (II) 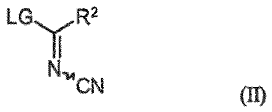 dans laquelle
R² et LG ont les significations indiquées précédemment,
puis ceux-ci sont mis en réaction lors d'une deuxième étape de réaction avec des composés de formule (VIII)
A-B-NH₂ (VIII)
dans laquelle
A et B ont les significations indiquées précédemment,
éventuellement en présence d'un diluant approprié et éventuellement en présence d'un adjuvant basique, pour former des composés de formule (IX) 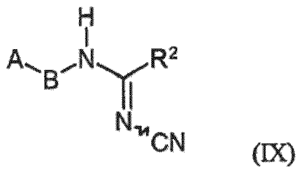 dans laquelle
A, B, R² ont les significations indiquées précédemment,
puis ceux-ci sont mis en réaction lors d'une troisième étape de réaction avec des composés de formule (X)
R¹-E (X)
dans laquelle
R¹ et E ont les significations indiquées précédemment,
éventuellement en présence d'un diluant approprié et éventuellement en présence d'un adjuvant basique.

4. Composé de formule (I) selon la revendication 1, dans lequel le composé de formule (I) est le composé de formule (I-1)

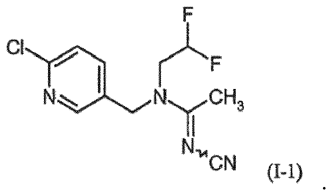

5. Composé de formule (IV-1)

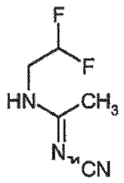

6. Agent, **caractérisé par** une teneur en au moins un composé de formule (I) selon la revendication 1 et des extendeurs et/ou tensioactifs usuels.

7. Procédé non thérapeutique de lutte contre des animaux nuisibles, **caractérisé en ce qu'**un composé de formule (I) selon la revendication 1 ou un agent selon la revendication 6 est laissé agir sur les animaux nuisibles et/ou leur habitat.

8. Utilisation de composés de formule (I) selon la revendication 1 ou d'agents selon la revendication 6 pour la lutte non thérapeutique contre des animaux nuisibles.
